(19) Europäisches Patentamt
European Patent Office
Office européen des brevets

(11) Veröffentlichungsnummer : **0 327 504 B1**

# (12) EUROPÄISCHE PATENTSCHRIFT

(45) Veröffentlichungstag der Patentschrift : **12.05.93 Patentblatt 93/19**

(51) Int. Cl.$^5$ : **C07C 275/54,** A01N 47/34, C07C 217/84, C07C 215/76, C07C 205/26, C07C 205/37

(21) Anmeldenummer : **89810075.5**

(22) Anmeldetag : **27.01.89**

## (54) N-Benzoyl-N'-trifluorphenylharnstoffe, ihre Herstellung und Verwendung bei der Kontrolle von Schädlingen.

Die Akte enthält technische Angaben, die nach dem Eingang der Anmeldung eingereicht wurden und die nicht in dieser Patentschrift enthalten sind.

(30) Priorität : **04.02.88 CH 394/88**
**01.12.88 CH 4456/88**

(43) Veröffentlichungstag der Anmeldung : **09.08.89 Patentblatt 89/32**

(45) Bekanntmachung des Hinweises auf die Patenterteilung : **12.05.93 Patentblatt 93/19**

(84) Benannte Vertragsstaaten : **AT BE CH DE ES FR GB GR IT LI NL**

(56) Entgegenhaltungen :
**EP-A- 0 161 019**
**EP-A- 0 194 688**

(56) Entgegenhaltungen :
**EP-A- 0 231 152**
**EP-A- 0 243 790**
**WO-A-86/05487**
**CHEMICAL ABSTRACTS, Band 89, Nr. 5, Seite 495, Spalte 2, 31. Juli 1978, Referat Nr. 41807z, Columbus, Ohio, US; R. BOLTON et al: "Nucleophilic displacement in polyhaloaromatic compounds. Part 7. Kinetics of methoxydefluorination of polyfluoronitrobenzenes", Formelregister Band 89, 1978, Seite 227F, Spalte 2, Zeilen 59-61**
**PATENT ABSTRACTS OF JAPAN, Band 11, Nr. 139 (C-420)(2586), 7. Mai 1987; & JP-A-61 277 660**

(73) Patentinhaber : **CIBA-GEIGY AG**
**Klybeckstrasse 141**
**CH-4002 Basel (CH)**

(72) Erfinder : **Drabek, Jozef, Dr.**
**Benkenstrasse 12**
**CH-4104 Oberwil (CH)**

EP 0 327 504 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue substituierte N-Benzoyl-N'-2,3,5-trifluor-4-alkoxy- oder halogenalkoxyphenylharnstoffe, Verfahren und Zwischenprodukte zu ihrer Herstellung, Schädlingsbekämpfungsmittel, die diese Verbindungen enthalten, sowie ihre Verwendung bei der Kontrolle von Schädlingen.

Die erfindungsgemässen Verbindungen entsprechen der Formel I

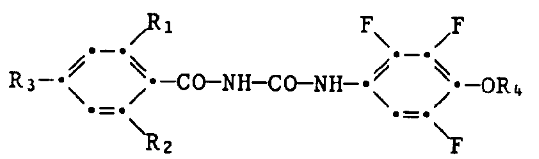

(I),

worin $R_1$ für Wasserstoff, Fluor oder Chlor; $R_2$ für Fluor oder Chlor; $R_3$ für Wasserstoff oder Fluor; und $R_4$ für $C_1$-$C_{10}$-Alkyl, -C(X)F-$C_1$-$C_7$-Alkyl oder ein- oder mehrfach durch Halogen substituiertes -C(X)F-$C_1$-$C_7$-Alkyl stehen, wobei X Wasserstoff oder Halogen bedeutet.

Je nach Art des Substituenten $R_4$ können die Verbindungen der Formel I auch als optisch aktive Isomere vorliegen. In solchen Fällen sind sowohl die reinen optisch aktiven Isomeren als auch deren Gemische Gegenstand der Erfindung.

Bei den als Substituenten in Betracht kommenden Halogenen handelt es sich sowohl um Fluor und Chlor als auch um Brom und Jod, wobei Fluor und Chlor bevorzugt sind.

Die als Substituenten in Betracht kommenden $C_1$-$C_{10}$-Alkyle können geradkettig oder verzweigt sein. Als Beispiele solcher Alkyle seien Methyl, Aethyl, Propyl, i-Propyl, Butyl, i-Butyl, sek.Butyl, tert.Butyl oder Pentyl, Hexyl, Heptyl, Octyl usw. und ihre Isomeren genannt.

Die als Substituenten in Betracht kommenden gegebenenfalls ein- oder mehrfach durch Halogen substituierten -C(X)F-$C_1$-$C_7$-Alkyle können geradkettig oder verzweigt und nur teilweise oder auch perhalogeniert sein, wobei für die Halogene und die Alkyle die oben gegebenen Definitionen gelten. Geeignete Beispiele solcher Substituenten sind u.a. das ein- bis fünffach durch Fluor, Chlor und/oder Brom substituierte Aethyl wie z.B. $CHFCF_3$, $CF_2CF_3$, $CF_2CCl_3$, $CF_2CHCl_2$, $CF_2CHF_2$, $CF_2CFCl_2$, $CF_2CHBr_2$, $CF_2CHClF$, $CF_2CHBrF$ oder $CClFCHClF$; das ein- bis siebenfach durch Fluor, Chlor und/oder Brom substituierte Propyl oder Isopropyl wie z.B. $CF_2CHBrCH_2Br$, $CF_2CHFCF_3$, $CHFCF_2CF_3$ oder $CHFCF_2CF_3$; das ein- bis neunfach durch Fluor, Chlor und/oder Brom substituierte Butyl oder eines seiner Isomeren wie z.B. $CHFCF_2CHFCF_3$ oder $(CF_2)_3CF_3$.

Unter den Verbindungen der Formel I stehen diejenigen im Vordergrund, in denen $R_1$ für Wasserstoff oder Fluor; $R_2$ für Fluor oder Chlor; $R_3$ für Wasserstoff oder Fluor; $R_4$ für $C_1$-$C_7$-Alkyl, -C(X)F-$C_1$-$C_5$-Alkyl oder ein- oder mehrfach durch Halogen substituiertes -C(X)F-$C_1$-$C_5$-Alkyl stehen, wobei X Wasserstoff, Fluor oder Chlor bedeutet.

Dabei sind diejenigen Verbindungen der Formel I bevorzugt, in denen $R_1$ für Wasserstoff oder Fluor; $R_2$ für Fluor oder Chlor; $R_3$ für Wasserstoff oder Fluor; und $R_4$ für $C_1$-$C_3$-Alkyl, -$CF_2$-$C_1$-$C_3$-Alkyl oder ein- oder mehrfach durch Fluor substituiertes -$CF_2$-$C_1$-$C_3$-Alkyl stehen.

Die erfindungsgemässen Verbindungen können nach an sich bekannten Verfahren hergestellt werden. Solche Verfahren sind u.a. in den DE-OS 21 23 236, 26 01 780 und 32 40 975 beschrieben. So können die Verbindungen der Formel I z.B. erhalten werden, indem man

a) ein Anilin der Formel II

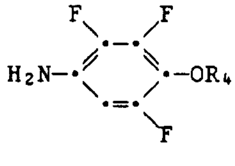

(II)

mit einem Benzoylisocyanat der Formel III

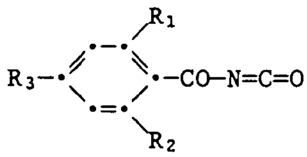

(III),

oder

b) ein Isocyanat der Formel IV

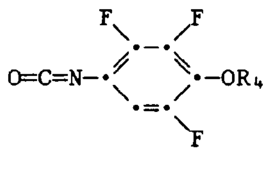

(IV)

mit einem Benzamid der Formel V

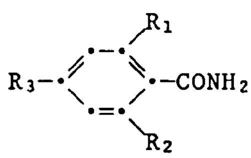

(V),

oder

c) ein Anilin der Formel II mit einem Urethan der Formel VI

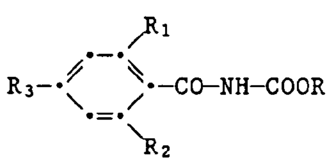

(VI)

umsetzt. In den Formeln III bis VI haben $R_1$, $R_2$, $R_3$ und $R_4$ die für Formel I angegebene Bedeutung und in Formel VI steht R für einen $C_1$-$C_8$-Alkylrest, der gegebenenfalls mit Halogen, vorzugsweise Chlor, substituiert sein kann.

Die erwähnten Verfahren a), b) und c) können vorzugsweise unter normalem Druck und in Gegenwart eines organischen Lösungs- oder Verdünnungsmittels durchgeführt werden. Als Lösungs- oder Verdünnungsmittel eignen sich z.B. Aether und ätherartige Verbindungen, wie Diäthyläther, Dipropyläther, Dibutyläther, Dioxan, Dimethoxyäthan und Tetrahydrofuran; N,N-dialkylierte Carbonsäureamide; aliphatische, aromatische sowie halogenierte Kohlenwasserstoffe, insbesondere Benzol, Toluol, Xylole, Chloroform, Methylenchlorid, Tetrachlorkohlenstoff und Chlorbenzol; Nitrile, wie Acetonitril oder Propionitril; Dimethylsulfoxid sowie Ketone, z.B. Aceton, Methyläthylketon, Methylisopropylketon und Methylisobutylketon. Verfahren a) wird im allgemeinen bei einer Temperatur von -10 bis +200°C, vorzugsweise zwischen 0 und +100°C, z.B. bei Raumtemperatur, gegebenenfalls in Gegenwart einer organischen Base, z.B. Triäthylamin, durchgeführt. Die Durchführung von Verfahren b) erfolgt bei einer Temperatur von 0 bis +150°C, vorzugsweise beim Siedepunkt des verwendeten Lösungsmittels, und gegebenenfalls in Gegenwart einer organischen Base, wie Pyridin, und/oder unter Zusatz eines Alkali-oder Erdalkalimetalls, vorzugsweise Natrium. Für das Verfahren c), d.h. für die Umsetzung der Urethane der Formel VI mit dem Anilin der Formel II, werden Temperaturen zwischen etwa +60°C und dem Siedepunkt des jeweiligen Reaktionsgemisches bevorzugt, wobei als Lösungsmittel insbesondere aromatische Kohlenwasserstoffe, wie Toluol, Xylole, Chlorbenzol usw., verwendet werden.

Die Ausgangsstoffe der Formeln III bis VI sind bekannt oder können analog bekannten Verfahren hergestellt werden.

Beim Ausgangsstoff der Formel II handelt es sich um neue Verbindungen, die ebenfalls einen Gegenstand der vorliegenden Erfindung bilden. Die Verbindungen der Formel II können in an sich bekannter Weise z.B.

dadurch hergestellt werden, dass man des Aminophenol der Formel VII

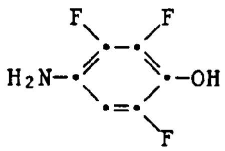

(VII)

nach im Prinzip bekannten Verfahren durch Addition von fluorierten Alkenylen oder ein- oder mehrfach durch Halogen substituierten Alkenylen der Formel $CF_2$=CF-$C_1$-$C_6$-Alkyl halogenalkyliert. Die Reaktion wird vorzugsweise in einem organischen Lösungsmittel wie z.B. Dimethylformamid bei normalem oder leicht erhöhtem Druck, bei einer Temperatur von -30 bis +150°C, vorzugsweise -10 bis +50°C und gegebenenfalls in Gegenwart eines basischen Katalysators wie z.B. Kaliumhydroxid, Natriumhydroxid, Triäthylamin oder Tetraäthylammoniumhydroxid durchgeführt (vgl. J. Am. Chem. Soc. 73, 1951, p. 5831; U.S. Patentschrift 3,937,726).

Das Aminophenol der Formel VII ist neu, bildet ebenfalls einen Gegenstand der vorliegenden Erfindung und kann nach einem im Prinzip bekannten Verfahren hergestellt werden, indem man z.B. das Nitrophenol der Formel VIII

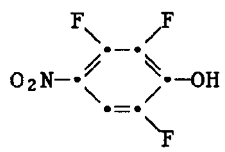

(VIII)

katalytisch hydriert. Als Katalysatoren kommen die üblichen Hydrierungskatalysatoren in Betracht wie z.B. Platin, Palladium, Rhenium, Rhenium-oxide oder Raney-Nickel (vgl. J. org. Chem. 29, 1964). Die Ueberführung der $NO_2$- in die $NH_2$-Gruppe kann auch durch übliche chemische Reduktion erfolgen z.B. mit Eisen oder Zinnchlorid in salzsaurem Medium.

Das Nitrophenol der Formel VIII ist aus J. Chem. Soc. Perkin Trans. 2, 1978(2), 141-144, bekannt und kann nach einem im Prinzip bekannten Verfahren hergestellt werden, indem man z.B. aus dem Alkoxynitrobenzol der Formel IX

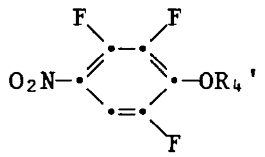

(IX),

worin $R_4$' für $C_1$-$C_{10}$-Alkyl steht, in stark saurem Medium z.B. in Halogenwasserstoffsäuren wie Salzsäure oder Bromwasserstoffsäure, die gegebenenfalls in Essigsäure oder einem anderen organischen Lösungsmittel gelöst sein können, bei +30 bis 130°C, vorzugsweise +80 bis 120°C die Alkylgruppe abspaltet (vgl. Chem. Rev. 54, 1954, pp. 615-685).

Die Alkoxynitrobenzole der Formel (IX), worin $R_4'$ für $C_2$-$C_{10}$-Alkyl steht, sind neu, bilden ebenfalls einen Gegenstand der vorliegenden Erfindung und können nach im Prinzip bekannten Verfahren hergestellt werden, indem z.B. das Nitrobenzol der Formel (X)

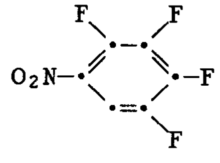

(X)

mit einem Metallalkoholat der Formel $MetOR_4$', worin Met für ein Erdalkali- vorzugsweise ein Alkalimetall steht und $R_4$' die oben angegebene Bedeutung hat, umsetzt.

Das Nitrobenzol der Formel X ist bekannt und kann nach im Prinzip bekannten Methoden hergestellt werden.

In den veröffentlichten europäischen Patentanmeldungen 0 071 279 und 0 243 790 werden ganz allgemein u.a. N-Halogenbenzoyl-N'-(halogen-4-halogenalkoxyphenyl)-harnstoffe als Insektizide mit larvizider Wirkung beschrieben. In diesen Veröffentlichungen werden jedoch keine N-(2-Mono- oder 2,6-Dihalogenbenzoyl)-N'-(2,3,5-trifluor-4-alkoxy-oder halogenalkoxyphenyl)-harnstoffe spezifisch offenbart.

Ueberraschenderweise wurde gefunden, dass die erfindungsgemässen Verbindungen der Formel I bei günstiger Warmblüter- und Pflanzenverträglichkeit stärkere Wirkstoffe in der Schädlingsbekämpfung sind. So eignen sich die Verbindungen der Formel I z.B. zur Bekämpfung von Schädlingen an Tieren und Pflanzen. Solche Schädlinge gehören hauptsächlich dem Stamm der Arthropoden an, wie insbesondere Insekten der Ordnungen Lepidoptera, Coleoptera, Homoptera, Heteroptera, Diptera, Thysanoptera, Orthoptera, Anoplura, Siphonaptera, Mallophaga, Thysanura, Isoptera, Psocoptera oder Hymenoptera und Arachniden der Ordnung Acarina, wie z.B. Milben und Zecken. Dabei kann jedes Entwicklungsstadium der Schädlinge bekämpft werden, d.h. sowohl die Adulten, Puppen und Nymphen, als auch insbesondere die Larven und Eier. So können vor allem Larven und Eier von phytopathogenen Schadinsekten und -milben in Zier- und Nutzpflanzungen, wie z.B. in Obst- und Gemüsepflanzungen, und insbesondere in Baumwollpflanzungen, wirkungsvoll bekämpft werden. Werden Verbindungen der Formel I von Imagines aufgenommen, so kann sich ihre Wirkung in unmittelbar Abtötung der Schädlinge zeigen oder aber in verminderter Eiablage und/oder Schlupfrate. Die letztere Erscheinung kann insbesondere bei Coleopteren beobachtet werden. Bei der Bekämpfung von tierparasitären Schädlingen, insbesondere an Haus- und Nutztieren, kommen vor allem Ektoparasiten, wie z.B. Milben und Zecken und Dipteren, wie z.B. Lucilia sericata in Betracht. Ausserdem zeigen die erfindungsgemässen Verbindungen eine bemerkenswerte Wirkung gegen phytophage Schnecken.

Insbesondere zeichnen sich die erfindungsgemässen Verbindungen durch eine ausgezeichnete larvizide Wirkung bei Spodoptera littoralis und vor allem bei Heliothis virescens aus.

Die gute pestizide Wirkung der erfindungsgemässen Verbindungen der Formel I entspricht einer Abtötungsrate (Mortalität) von mindestens 50-60 % der erwähnten Schädlinge.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen. Als Zusätze kommen z.B. Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formel I werden in unveränderter Form oder vorzugsweise zusammen mit den in der Formulierungstechnik üblichen, inerten, pflanzenverträglichen Hilfsmitteln eingesetzt und können daher z.B. zu Emulsionskonzentraten, direkt versprüh- oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet werden. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, d.h. die einen Wirkstoff der Formel I, bzw. Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen nichtionogene, kation- und/oder anionaktive Tenside mit guten Emulgier-, Dispergier- und Netzeigenschaften in Betracht. Unter Tensiden sind auch Tensidgemische zu

verstehen.

Geeignete anionische Tenside können sowohl sogenannte wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Tallöl gewonnen werden können. Ferner sind als Tenside auch die Fettsäure-methyl-taurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie z.B. Salze des Phosphorsäureesters eines p-Nonylphenol-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykol-Einheiten.

Als Beispiele nichtionischer Tenside seien Nonylphenolpolyäthoxyäthanole, Ricinusölpolyglykoläther, Ricinusölthioxilat, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogeniertes Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben:

"Mc Cutcheon's Detergents and Emulsifiers Annual" MC Publishing Corp., Ridgewood, New Jersey, 1979;

Dr. Helmut Stache "Tensid Taschenbuch", Carl Hanser Verlag München/Wien 1981.

Die pestiziden Zubereitungen enthalten in der Regel 0,1 bis 99 %, insbesondere 0,1 bis 95 %, Wirkstoff der Formel I oder Kombinationen dieser Wirkstoffe mit andern Insektiziden oder Akariziden, 1 bis 99,9 % eines festen oder flüssigen Zusatzstoffes und 0 bis 25 %, insbesondere 0,1 bis 20 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Beispiel 1: Herstellung

1.1. Zwischenprodukte

1.1.1. Alkoxynitrobenzole

1.1.1.1. 2,3,5-Trifluor-4-äthoxy-nitrobenzol

17,93 g 2,3,4,5-Tetrafluor-nitrobenzol und 25 ml Aethanol werden tropfenweise unter Eiskühlung mit einer Lösung von 2,3 g Natrium in 50 ml absolutem Aethanol versetzt und eine Stunde lang bei +10°C nachgerührt.

Anschliessend wird das Reaktionsgemisch in 300 ml Eis/Wasser gegossen, mit Aether extrahiert und die Aetherphase über Natriumsulfat getrocknet. Das Lösungsmittel wird am Vacuum abdestilliert. Die Titelverbindung der Formel

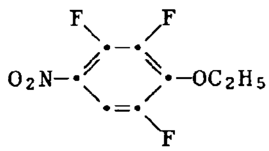

(Verb. Nr. 1.1.1.1.)

liegt als gelbe Flüssigkeit vor; Sdp. 57-60°C/0,07 mbar.

Auf analoge Weise werden die folgenden Verbindungen hergestellt:

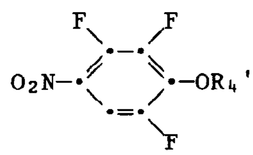

| Verb. Nr. | $R_4$' | physikalische Daten |
|---|---|---|
| 1.1.1.2. | $(CH_2)_2CH_3$ | Sdp. 77-83°C/0,053 mbar |
| 1.1.1.3. | $CH(CH_3)_2$ | Sdp. 76°C/0,027 mbar |
| 1.1.1.4. | $(CH_2)_3CH_3$ | Sdp. 80-83°C/0,027 mbar |
| 1.1.1.5. | $C(CH_3)_3$ | Sdp. 62-67°C/0,013 mbar |
| | $CH_3$ | |

1.1.2. 2,3,6-Trifluor-4-nitrophenol

14 g 2,3,5-Trifluor-4-äthoxy-nitrobenzol, 400 ml 40%ige Bromwasserstoffsäure und 400 ml Essigsäure werden 8 Stunden lang unter Rühren auf Rückflusstemperatur erhitzt. Anschliessend werden Essigsäure, Wasser und Bromwasserstoffsäure abdestilliert und der Rückstand auf Eiswasser gegossen. Der entstandene Niederschlag wird abgenutscht und aus Hexan umkristallisiert. Die Titelverbindung der Formel

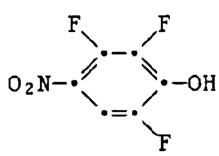

(Verb. Nr. 1.1.2.)

liegt in Form hellgelber Kristalle vor; Smp. 110-112°C.

1.1.3. Aniline

1.1.3.1. 2,3,6-Trifluor-4-aminophenol

19,8 g 2,3,6-Trifluor-4-nitrophenol werden in 200 ml Tetrahydrofuran gelöst und bei +30 bis 35°C während 4 1/2 Stunden in Gegenwart von 6 g Raney-Nickel hydriert. Das Reaktionsgemisch wird filtriert und das Lösungsmittel abdestilliert und der Rückstand aus Toluol/Hexan umkristallisiert. Die Titelverbindung der Formel

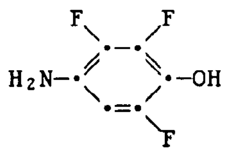

(Verb. Nr. 1.1.3.1.)

liegt in Form farbloser Kristalle vor; Smp. 133-135°C.

1.1.3.2. Alkoxyaniline

1.1.3.2.1. 2,3,5-Trifluor-4-äthoxyanilin

9,7 g 2,3,5-Trifluor-4-äthoxynitrobenzol werden in 100 ml Tetrahydrofuran gelöst und bei +30 bis 35°C während 9 1/2 Stunden in Gegenwart von Raney-Nickel hydriert. Das Reaktionsgemisch wird filtriert, das Lösungsmittel abdestilliert und der Rückstand am Vacuum destilliert. Die Titelverbindung der Formel

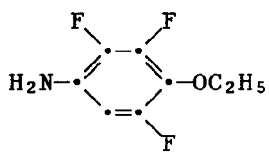

(Verb. Nr. 1.1.3.2.1.

liegt als farblose Flüssigkeit vor; Sdp. 100°C/0,027 mbar.

Auf analoge Weise werden die folgenden Verbindungen hergestellt:

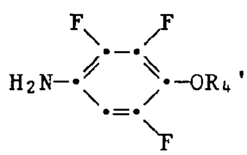

| Verb. Nr. | $R_4$' | physikalische Daten |
|---|---|---|
| 1.1.3.2.2. | $(CH_2)_2CH_3$ | Sdp. 100°C/0,013 mbar |
| 1.1.3.2.3. | $CH(CH_3)_2$ | Sdp. 110°C/0,066 mbar |
| 1.1.3.2.4. | $(CH_2)_3CH_3$ | Sdp. 110°C/0,053 mbar |
| 1.1.3.2.5. | $C(CH_3)_3$ | Sdp. 100°C/0,040 mbar |
| | $CH_3$ | |

1.1.3.3. Halogenalkoxyaniline

1.1.3.3.1. 2,3,5-Trifluor-4-(1,1,2,3,3,3-hexafluorpropoxy)-anilin

15 g Hexafluorpropylen werden unter Rühren in eine Lösung von 13,4 g 2,3,5-Trifluor-4-aminophenol und 1,2 ml Triäthylamin in 80 ml Dimethylformamid eingeleitet und das Reaktionsgemisch 2 Stunden lang bei Raumtemperatur nachgerührt. Anschliessend wird das Reaktionsgemisch eingeengt und der Rückstand am Vakuum destilliert. Die Titelverbindung der Formel

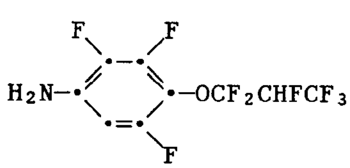

(Verb. 1.1.3.3.1.)

liegt als farblose Flüssigkeit vor; Sdp. 59-62°C/0,013 mbar.

Auf analoge Weise werden die folgenden Verbindungen hergestellt:

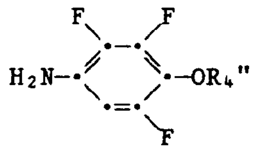

| Verb. Nr. | $R_4''$ | physikalische Daten |
|---|---|---|
| 1.1.3.3.2. | $CF_2CHF_2$ | Sdp. 130°C/0,053 mbar |
| 1.1.3.3.3. | $CF_2CHClF$ | Sdp. 125°C/0,040 mbar |
| | $(CF_2)_2CF_3$ | |
| | $(CF_2)_6CF_3$ | |
| | $CHFCHFCF_3$ | |

## 1.2. Endprodukte

### 1.2.1. N-(2,6-Difluorbenzoyl)-N'-[2,3,5-trifluor-4-(1',1',2',3',3',3'-hexafluorpropoxy)-phenyl]-harnstoff

5,17 g 2,3,5-Trifluor-4-(1',1',2',3',3',3'-hexafluorpropoxy)-anilin, gelöst in 50 ml trockenem Toluol, werden bei Raumtemperatur mit 3 g 2,6-Difluorbenzoylisocyanat versetzt und 5 Stunden lang gerührt. Anschliessend werden etwa 70 % des Lösungsmittels am Rotationsverdampfer entfernt. Der entstandene Niederschlag wird abgenutscht, mit wenig kaltem Hexan gewaschen und im Vakuum getrocknet. Die Titelverbindung der Formel

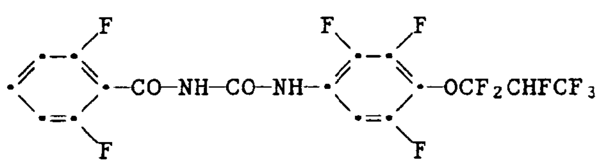

(Verb. Nr. 1.2.1.)

liegt als farbloses kristallines Pulver vor; Smp. 169-171°C.

Auf analoge Weise werden die folgenden Verbindungen hergestellt:

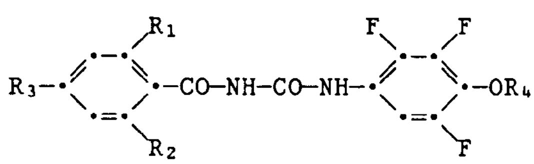

| Verb. Nr. | $R_1$ | $R_2$ | $R_3$ | $R_4$ | phys. Daten |
|---|---|---|---|---|---|
| 1.2.2. | H | Cl | H | $CF_2CHFCF_3$ | Smp. 149-151°C |
| 1.2.3. | F | F | H | $CH_2CH_3$ | Smp. 194-196°C |
| 1.2.4. | H | Cl | H | $CH_2CH_3$ | Smp. 177-178°C |
| 1.2.5. | F | F | H | $(CH_2)_2CH_3$ | Smp. 190-191°C |
| 1.2.6. | H | Cl | H | $(CH_2)_2CH_3$ | Smp. 152-155°C |
| 1.2.7. | F | F | H | $CF_2CHClF$ | Smp. 170-172°C |
| 1.2.8. | F | F | H | $CF_2CHF_2$ | Smp. 167-168°C |
| 1.2.9. | F | F | H | $CH(CH_3)_2$ | Smp. 172-174°C |
| 1.2.10. | F | F | H | $(CH_2)_3CH_3$ | Smp. 159-161°C |
| 1.2.11. | H | F | H | $CF_2CHFCF_3$ | Smp. 148-150°C |
| 1.2.12. | F | Cl | H | $CF_2CHFCF_3$ | Smp. 198-200°C |
| 1.2.13. | F | F | H | $C(CH_3)_3$ | Smp. 218-219°C |
| 1.2.14. | H | Cl | H | $C(CH_3)_3$ | Smp. 178-179°C |
| 1.2.15. | H | Cl | H | $(CH_2)_3CH_3$ | Smp. 133-135°C |
| 1.2.16. | H | Cl | H | $CH(CH_3)_2$ | Smp. 159-160°C |
| 1.2.17. | F | Cl | H | $(CH_2)_3CH_3$ | Smp. 150-152°C |
| 1.2.18. | H | F | H | $(CH_2)_3CH_3$ | Smp. 153-154°C |
| 1.2.19. | F | Cl | H | $CH\ (CH_3)_2$ | Smp. 206-208°C |
| 1.2.20. | F | F | F | $CF_2CHFCF_3$ | Smp. 187-188°C |
| 1.2.21. | F | F | F | $CF_2CHF_2$ | Smp. 184-186°C |
| 1.2.22. | F | F | F | $CF_2CHClF$ | Smp. 185-187°C |
| | F | F | F | $CF_2CHFCF_3$ | |
| | F | F | H | $(CF_2)_2CF_3$ | |
| | F | F | H | $(CF_2)_6CF_3$ | |
| | F | F | F | $CH_3$ | |
| | F | F | H | $(CH_2)_9CH_3$ | |
| | Cl | Cl | F | $CH_2CH_3$ | |
| | F | Cl | F | $CHFCHFCHF_2$ | |

Beispiel 2: Formulierungen von Wirkstoffen der Formel I gemäss Herstellungsbeispiel 1.2.

(% = Gewichtsprozent)

| 2.1. Emulsions-Konzentrate | a) | b) |
|---|---|---|
| Wirkstoff gemäss dem Herstellungsbeispiel 1.2. | 5 % | 12 % |
| K-Dodecylbenzolsulfonat | 4 % | 3 % |
| Ricinusöl-polyäthylenglykoläther (36 Mol AeO) | 6 % | 12 % |
| Cyclohexanon | 20 % | - |
| Xylolgemisch | 65 % | 48 % |
| N-Methyl-2-pyrrolidon | - | 20 % |
| Dimethylcyclohexylphthalat | - | 5 % |

Aus solchen Konzentraten können durch Verdünnen mit Wasser Emulsionen jeder gewünschten Konzentration hergestellt werden.

| 2.2. Lösungen | a) | b) |
|---|---|---|
| Wirkstoff gemäss dem Herstellungsbeispiel 1.2. | 10 % | 5 % |
| Polyäthylenglykol (MG 400) | 70 % | - |
| N-Methyl-2-pyrrolidon | 20 % | 20 % |
| Epoxidiertes Kokosnussöl | - | 1 % |
| Benzin (Siedegrenzen 160-190°C) | - | 74 % |

Die Lösungen sind zur Anwendung in Form kleinster Tropfen geeignet.

| 2.3. Granulate | a) | b) |
|---|---|---|
| Wirkstoff gemäss dem Herstellungsbeispiel 1.2. | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 2.4. Extruder Granulat | |
|---|---|
| Wirkstoff gemäss dem Herstellungsbeispiel 1.2. | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| 2.5. Umhüllungs-Granulat | |
|---|---|
| Wirkstoff gemäss dem Herstellungsbeispiel 1.2. | 3 % |
| Polyäthylenglykol (MG 200) | 3 % |
| Kaolin | 94 % |

Der feingemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| 2.6. Stäubemittel | a) | b) | c) | d) |
|---|---|---|---|---|
| Wirkstoff gemäss dem Herstellungsbeispiel 1.2. | 2 % | 5 % | 5 % | 8 % |
| Hochdisperse Kieselsäure | 1 % | 5 % | - | - |
| Talkum | 97 % | - | 95 % | - |
| Kaolin | - | 90 % | - | 92 % |

Durch inniges Vermischen der Trägerstoffe mit dem Wirkstoff und gegebenenfalls Vermahlen auf einer geeigneten Mühle erhält man gebrauchsfertige Stäubemittel.

| 2.7. Spritzpulver | a) | b) | c) |
|---|---|---|---|
| Wirkstoff gemäss dem Herstellungsbeispiel 1.2. | 20 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 67 % | 27 % | - |

Der Wirkstoff wird mit den Zusatzstoffen gut vermischt und in einer geeigneten Mühle vermahlen. Man erhält Spritzpulver, die sich mit Wasser zu Suspensionen jeder gewünschten Konzentration verdünnen lassen.

2.8. Suspensions-Konzentrat

| | |
|---|---|
| Wirkstoff gemäss dem Herstellungsbeispiel 1.2. | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 3: Biologische Prüfungen

3.1. Wirkung gegen Musca domestica

Ein Zuckerwürfel wird derart mit einer Lösung der Testsubstanz befeuchtet, dass die Konzentration des Wirkstoffes im Würfel nach dem Trocknen 500 ppm beträgt. Der so behandelte Würfel wird zusammen mit einem nassen Wattebausch auf eine Schale gelegt und mit einem Becherglas zugedeckt. Unter das Becherglas werden 10 adulte, eine Woche alte und OP-resistente Fliegen gegeben und bei 25°C und 50 % Luftfeuchtigkeit belassen. Nach 24 Stunden wird die insektizide Wirkung durch Ermittlung der Abtötungsrate festgestellt.

Verbindungen gemäss Beispiel 1.2. zeigen gute Wirkung im obigen Test.

3.2. Wirkung gegen Schmeissfliegen

Frisch abgelegte Eier der Schmeissfliegenart Lucilia sericata werden in kleinen Portionen (30-50 Eier) in Reagenzgläser gegeben, in denen zuvor 4 ml Nährmedium mit 1 ml Testlösung, die 200 ppm des zu prüfenden Wirkstoffes enthält, vermischt worden sind. Nach Beimpfung des Kulturmediums werden die Testgläser mit einem Wattestopfen verschlossen und im Brutschrank bei 30°C 4 Tage lang bebrütet. Im unbehandelten Medium entwickeln sich bis zu diesem Zeitpunkt ca. 1 cm grosse Larven (Stadium 3). Ist diese Substanz aktiv, so sind die Larven zu diesem Zeitpunkt entweder tot oder deutlich zurückgeblieben. Die Auswertung erfolgt nach 96 Stunden; es wird die Mortalität in Prozenten bestimmt.

Verbindungen gemäss Beispiel 1.2. zeigen gute larvizide Wirkung.

3.3. Wirkung gegen Aedes aegypti

Auf die Oberfläche von 150 ml Wasser, das sich in einem Behälter befindet, wird so viel einer 0,1%igen acetonischen Lösung des Wirkstoffes pipettiert, dass eine Konzentration von 12,5 ppm erhalten wird. Nach Verdunsten des Acetons wird der Behälter mit 30-40 2tägigen Aedes-Larven beschickt. Nach 2 und 7 Tagen wird die Mortalität geprüft.

Verbindungen gemäss Beispiel 1.2. zeigen gute Wirkung in diesem Test.

3.4. Frassgift-Wirkung auf Spodoptera littoralis-Larven ($L_1$)

Baumwollpflanzen im Keimblattstadium werden mit einer wässrigen Wirkstoffemulsion (erhalten aus einem 10%igen emulgierbaren Konzentrat) besprüht, wobei die Wirkstoffemulsion 400 ppm der zu prüfenden Verbindung enthält.

Nach dem Antrocknen des Belages wird jede Baumwollpflanze mit Spodoptera littoralis-Larven im ersten larvalen Stadium besetzt. Der Versuch wird bei 26°C und ca. 50 % relativer Luftfeuchtigkeit durchgeführt. Nach

2 und 3 Tagen wird die Mortalität und nach 5 Tagen werden Entwicklungs- und Häutungsstörungen der angesetzten Larven bestimmt.

Verbindungen gemäss Beispiel 1.2. zeigen 100%ige Wirkung.

3.5. Frassgift-Wirkung auf Spodoptera littoralis- und Heliothis virescens-Larven ($L_3$)

Eingetopfte Sojapflanzen (Topfgrösse 10 cm Durchmesser) im 4-Blatt-Stadium werden mit wässrigen Wirkstoffemulsionen besprüht, die den Wirkstoff in einer Konzentration von 0,75 ppm enthalten.

Nach zwei Tagen werden die behandelten Sojapflanzen mit je 10 Larven von Spodoptera littoralis und Heliothis virescens im dritten larvalen Stadium besiedelt. Der Versuch wird bei 26°C und ca. 60 % relativer Luftfeuchtigkeit im Dämmerlicht durchgeführt. Die Bonitur erfolgt nach 2 und 5 Tagen; es wird die Mortalität der Larven in Prozenten bestimmt.

Verbindungen gemäss Beispiel 1.2. zeigen bei Spodoptera und bei Heliothis nach 2 und 5 Tagen 80-100%ige Wirkung (Mortalität).

3.6. Frassgift-Wirkung auf Plutella xylostella-Larven ($L_2$)

Eingetopfte Chinakohlpflanzen (Topfgrösse 10 cm Durchmesser) im 4-Blatt-Stadium werden mit einer wässrigen Wirkstoffemulsion besprüht, die den Wirkstoff in einer Konzentration von 0,75 ppm enthält.

Nach zwei Tagen werden die behandelten Chinakohlpflanzen mit 10 Plutella xylostella-Larven im zweiten larvalen Stadium besiedelt. Der Versuch wird bei 26°C und ca. 60% relativer Luftfeuchtigkeit im Dämmerlicht durchgeführt. Die Bonitur erfolgt nach 2 und 5 Tagen; es wird die Mortalität der Larven in Prozenten bestimmt.

Verbindungen gemäss Beispiel 1.2. zeigen nach 2 und 5 Tagen 80-100%ige Wirkung (Mortalität).

3.7. Wirkung auf Laspeyresia pomonella (Eier)

Abgelegte Eier von Laspeyresia pomonella, die nicht älter als 24 Stunden sind, werden auf Filterpapier für 1 Minute in eine acetonisch-wässrige Lösung, enthaltend 400 ppm des zu prüfenden Wirkstoffes, eingetaucht. Nach dem Eintrocknen der Lösung wird das Filterpapier mit den Eiern in einer Petrischale ausgelegt und bei einer Temperatur von 28°C belassen. Nach 6 Tagen wird der prozentuale Schlupf aus den behandelten Eiern bewertet.

Verbindungen gemäss Beispiel 1.2. zeigen gute Wirkung in obigem Test.

3.8. Reproduktions-Beeinflussung von Anthonomus grandis

Adulte Anthonomus grandis, die nach dem Schlupf nicht älter als 24 Stunden sind, werden in Gruppen von jeweils 25 Käfern in Käfige mit Gitterwänden überführt. Die mit den Käfern besetzten Käfige werden sodann während 5 bis 10 Sekunden in eine acetonische Lösung, enthaltend 0,1 Gew.% des zu prüfenden Wirkstoffes, eingetaucht.

Wenn die Käfer wieder trocken sind, werden sie zur Kopulation und Eiablage in abgedeckte und Futter enthaltende Schalen eingesetzt. Abgelegte Eier werden zwei- bis dreimal wöchentlich mit fliessendem Wasser ausgeschwemmt, gezählt, durch zwei- bis dreistündiges Einlegen in ein wässriges Desinfektionsmittel desinfiziert und dann in Schalen, die eine geeignete Larvaldiät enthalten, deponiert. Nach 7 Tagen wird untersucht, ob sich aus den deponierten Eiern Larven entwickelt haben.

Zur Ermittlung der Dauer des die Reproduktion beeinflussenden Effektes der zu prüfenden Wirkstoffe wird die Eiablage der Käfer während eines Zeitraumes von etwa vier Wochen überprüft. Die Bonitierung erfolgt anhand der Verminderung der Anzahl abgelegter Eier und der daraus geschlüpften Larven im Vergleich zu unbehandelten Kontrollen.

Verbindungen gemäss Beispiel 1.2. zeigen eine gute reproduktionsreduzierende Wirkung im obigen Test.

3.9. Wirkung gegen Anthonomus grandis (Adulte)

Zwei eingetopfte Baumwollpflanzen im 6-Blattstadium werden mit wässrigen benetzungsfähigen Emulsions-Zubereitungen, enthaltend 100 ppm des zu prüfenden Wirkstoffes, besprüht. Nach dem Antrocknen des Spritzbelages (etwa 1,5 Stunden) wird jede Pflanze mit 10 adulten Käfern (Anthonomus grandis) besiedelt. Plastikzylinder, deren obere Oeffnungen mit Gaze abgedeckt sind, werden dann über die behandelten, mit den Testtieren besiedelten Pflanzen gestülpt, um ein Abwandern der Käfer zu verhindern. Die behandelten Pflanzen werden bei 25°C und etwa 60 % relativer Luftfeuchtigkeit gehalten. Die Auswertung erfolgt nach 2, 3, 4

und 5 Tagen unter Zugrundelegung der prozentualen Mortalität der eingesetzten Testtiere (% Rückenlage) sowie der Antifeedant-Wirkung gegenüber unbehandelten Kontrollansätzen.

Verbindungen gemäss Beispiel 1.2. zeigen gute Wirkung in diesem Test.

3.10. Wirkung gegen Epilachna varivestis

Etwa 15-20 cm hohe Phaseolus vulgaris-Pflanzen (Buschbohnen) werden mit wässrigen, den zu prüfenden Wirkstoff in einer Konzentration von 800 ppm enthaltenden Emulsions-Zubereitungen besprüht. Nach dem Antrocknen des Sprühbelages werden pro Pflanze 5 Larven von Epilachna varivestis (Mexikanischer Bohnenkäfer) im 4. larvalen Stadium angesetzt. Ueber die infestierten Pflanzen wird ein Plastikzylinder gestülpt, der mit einem Kupfer-Gazedeckel abgedeckt ist. Der Versuch wird bei 28°C und 60 % relativer Luftfeuchtigkeit durchgeführt.

Nach 2 und 3 Tagen wird die Mortalität in Prozenten bestimmt. Zur Auswertung hinsichtlich allfälligem Frassschaden (Antifeeding-Effekt), Entwicklungs- und Häutungsstörungen werden die Versuchstiere während weiterer 3 Tage beobachtet.

Verbindungen gemäss Beispiel 1.2. zeigen gute Wirkung im obigen Test.

3.11. Ovizide Wirkung auf Heliothis virescens und Spodoptera littoralis

Entsprechende Mengenanteile einer benetzbaren pulverförmigen Formulierung, enthaltend 25 Gew.% des zu prüfenden Wirkstoffes, werden mit jeweils so viel Wasser vermischt, dass sich eine wässrige Emulsion mit einer Wirkstoffkonzentration von 400 ppm ergibt.

In diese wirkstoffhaltige Emulsion werden eintägige auf Cellophan abgelegte Eigelege von Heliothis und auf Papier abgelegte Eigelege von Spodoptera während drei Minuten eingetaucht und dann auf Rundfilter abgenutscht. Die so behandelten Gelege werden in Petrischalen ausgelegt und bei 28°C und 60 % relativer Luftfeuchtigkeit in der Dunkelheit aufbewahrt. Nach 5 und 8 Tagen wird die Schlupfrate, d.h. die Anzahl Larven, die sich aus den behandelten Eiern entwickelt haben, gegenüber unbehandelten Kontrollansätzen bestimmt.

Verbindungen gemäss Beispiel 1.2. zeigen in obigem Test eine 80-100%ige ovizide Wirkung (Mortalität) gegen Heliothis virescens und Spodoptera littoralis.

## Patentansprüche

### Patentansprüche für folgende Vertragsstaaten : AT, BE, CH, DE, FR, GB, GR, IT, LI, NL

**1.** Verbindungen der Formel I

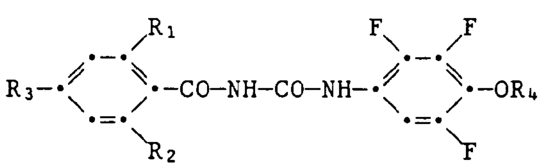

(I),

worin $R_1$ für Wasserstoff oder Fluor oder Chlor; $R_2$ für Fluor oder Chlor; $R_3$ für Wasserstoff oder Fluor; und $R_4$ für $C_1$-$C_{10}$-Alkyl, -C(X)F-$C_1$-$C_7$-Alkyl oder ein- oder mehrfach durch Halogen substituiertes -C(X)F-$C_1$-$C_7$-Alkyl stehen, wobei X Wasserstoff oder Halogen bedeutet.

**2.** Verbindungen der Formel I gemäss Anspruch 1, worin $R_1$ für Wasserstoff oder Fluor; $R_2$ für Fluor oder Chlor; $R_3$ für Wasserstoff oder Fluor; und $R_4$ für $C_1$-$C_7$-Alkyl, -C(X)F-$C_1$-$C_5$-Alkyl oder ein- oder mehrfach durch Halogen substituiertes -C(X)F-$C_1$-$C_5$-Alkyl stehen, wobei X Wasserstoff, Fluor oder Chlor bedeutet.

**3.** Verbindungen der Formel I gemäss Anspruch 2, worin $R_1$ für Wasserstoff oder Fluor; $R_2$ für Fluor oder Chlor; $R_3$ für Wasserstoff oder Fluor; und $R_4$ für $C_1$-$C_3$-Alkyl, -$CF_2$-$C_1$-$C_3$-Alkyl oder ein- oder mehrfach durch Fluor substituiertes -$CF_2$-$C_1$-$C_3$-Alkyl stehen.

**4.** Die Verbindungen gemäss einem der Ansprüche 1 bis 3 der Formeln

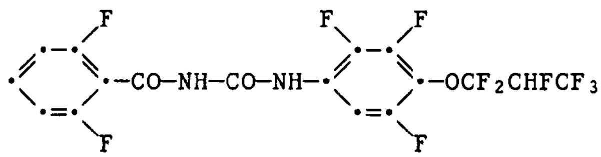 ,

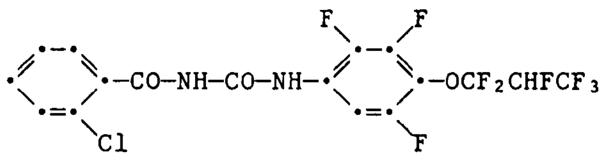 ,

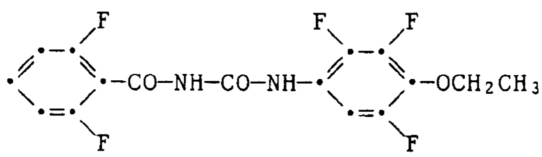 ,

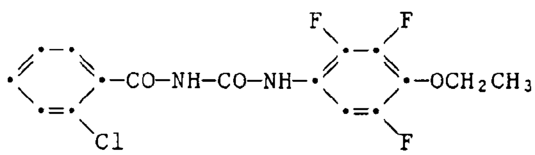 ,

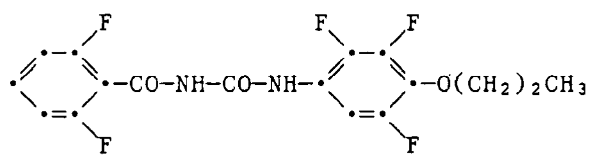 ,

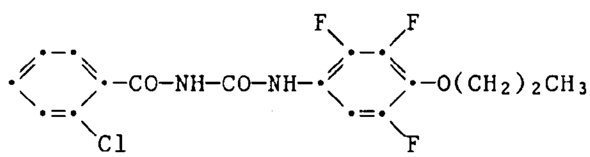 ,

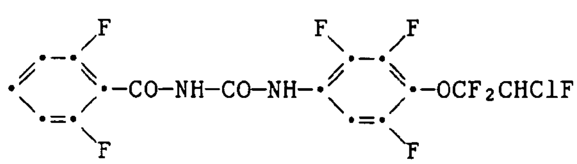 ,

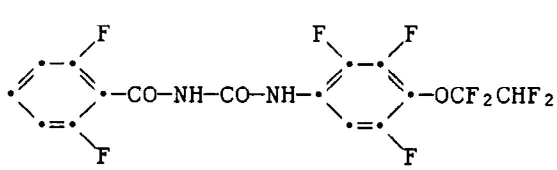 ,

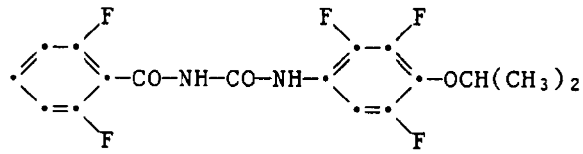,

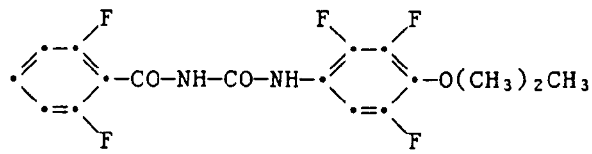,

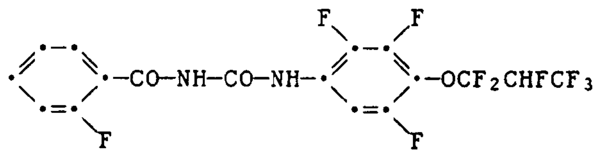,

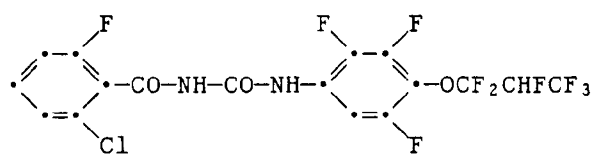,

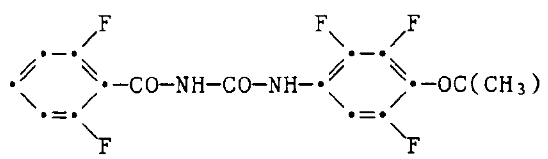,

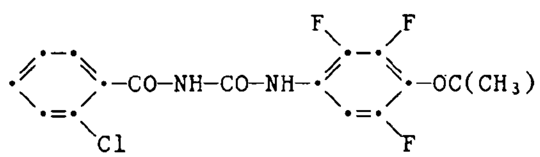,

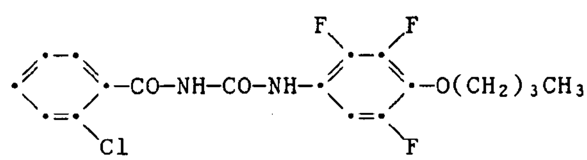,

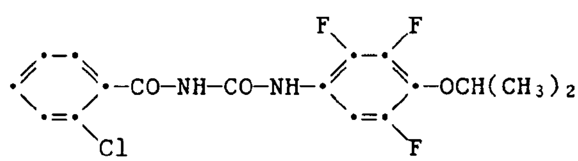,

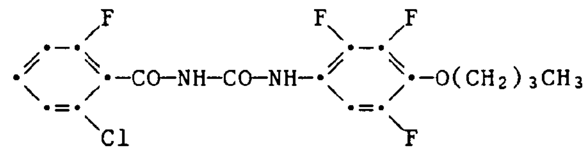
,

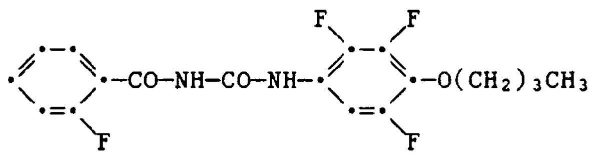
,

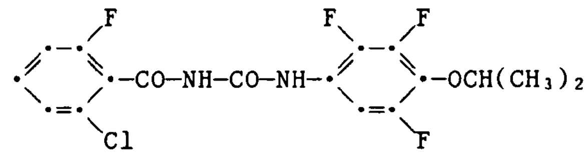
,

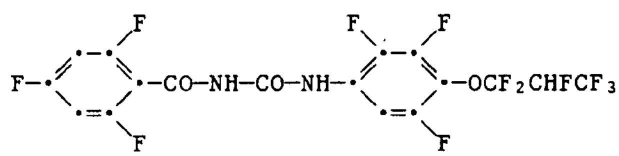
,

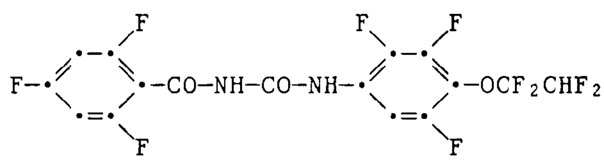

und

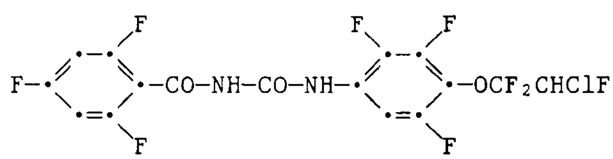
.

**5.** Verfahren zur Herstellung einer Verbindung der Formel I

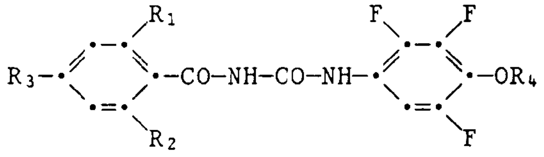
(I),

worin $R_1$ für Wasserstoff, Fluor oder Chlor; $R_2$ für Fluor oder Chlor; $R_3$ für Wasserstoff oder Fluor; und $R_4$ für $C_1$-$C_{10}$-Alkyl, -C(X)F-$C_1$-$C_7$-Alkyl oder ein- oder mehrfach durch Halogen substituiertes -C(X)F-$C_1$-$C_7$-Alkyl stehen, wobei X Wasserstoff oder Halogen bedeutet, dadurch gekennzeichnet, dass man

a) ein Anilin der Formel II

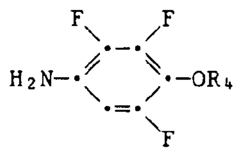

(II)

mit einem Benzoylisocyanat der Formel III

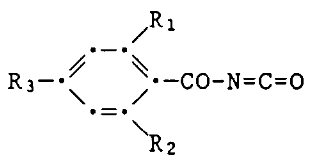

(III),

oder

b) ein Isocyanat der Formel IV

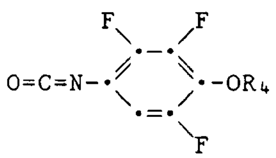

(IV)

mit einem Benzamid der Formel V

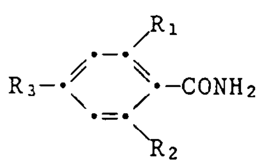

(V),

oder

c) ein Anilin der Formel II mit einem Urethan der Formel VI

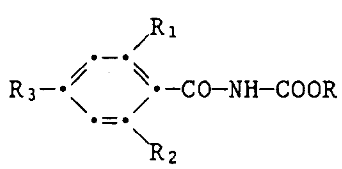

(VI)

umsetzt, wobei in den Formeln II, III, IV, V und VI $R_1$, $R_2$, $R_3$ und $R_4$ die angegebene Bedeutung haben und in Formel VI R für einen gegebenenfalls halogenierten $C_1$-$C_8$-Alkylrest steht.

**6.** Schädlingsbekämpfungsmittel, welches als aktive Komponente eine wirksame Menge einer Verbindung der Formel I

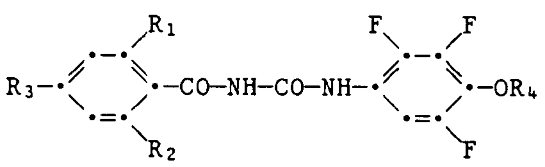

(I),

worin $R_1$ für Wasserstoff, Fluor oder Chlor; $R_2$ für Fluor oder Chlor; $R_3$ für Wasserstoff oder Fluor; und $R_4$ für $C_1$-$C_{10}$-Alkyl, -C(X)F-$C_1$-$C_7$-Alkyl oder ein- oder mehrfach durch Halogen substituiertes -C(X)F-$C_1$-$C_7$-

Alkyl stehen, wobei X Wasserstoff oder Halogen bedeutet, zusammen mit geeigneten Trägern und/oder Zuschlagstoffen enthält.

**7.** Verwendung einer Verbindung der Formel I

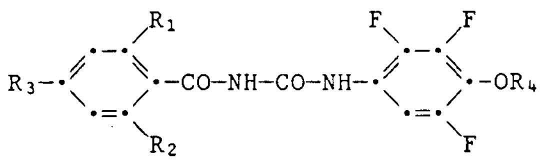

(I),

worin $R_1$ für Wasserstoff, Fluor oder Chlor; $R_2$ für Fluor oder Chlor; $R_3$ für Wasserstoff oder Fluor; und $R_4$ für $C_1$-$C_{10}$-Alkyl, -C(X)F-$C_1$-$C_7$-Alkyl oder ein- oder mehrfach durch Halogen substituiertes -C(X)F-$C_1$-$C_7$-Alkyl stehen, wobei X Wasserstoff oder Halogen bedeutet, zur Kontrolle von Schädlingen an Tieren und Pflanzen.

**8.** Verwendung gemäss Anspruch 7 einer Verbindung der Formel I zur Kontrolle von Insekten und Arachniden.

**9.** Verwendung gemäss Anspruch 8 einer Verbindung der Formel I zur Kontrolle larvaler Stadien von pflanzenschädigenden Insekten.

**10.** Verfahren zur Kontrolle von Schädlingen an Tieren und Pflanzen, dadurch gekennzeichnet, dass man die Schädlinge in ihren verschiedenen Entwicklungsstadien mit einer Verbindung der Formel I

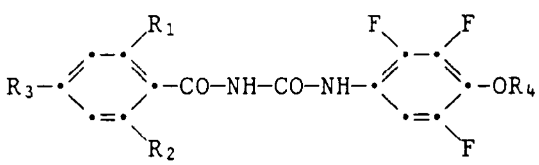

(I),

worin $R_1$ für Wasserstoff, Fluor oder Chlor; $R_2$ für Fluor oder Chlor; $R_3$ für Wasserstoff oder Fluor; und $R_4$ für $C_1$-$C_{10}$-Alkyl, -C(X)F-$C_1$-$C_7$-Alkyl oder ein- oder mehrfach durch Halogen substituiertes -C(X)F-$C_1$-$C_7$-Alkyl stehen, wobei X Wasserstoff oder Halogen bedeutet, in Kontakt bringt.

**11.** Verbindungen der Formel II

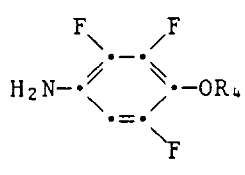

(II),

worin $R_4$ für $C_1$-$C_{10}$-Alkyl, -C(X)F-$C_1$-$C_7$-Alkyl oder ein- oder mehrfach durch Halogen substituiertes -C(X)F-$C_1$-$C_7$-Alkyl steht, wobei X Wasserstoff oder Halogen bedeutet.

**12.** Die Verbindungen gemäss Anspruch 11 der Formeln

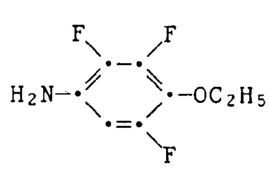

,

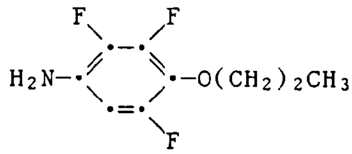
,

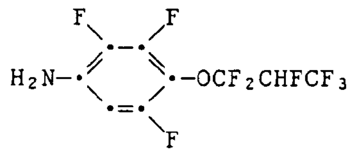
,

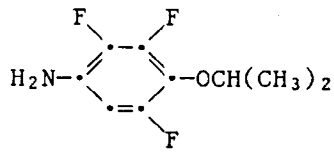
,

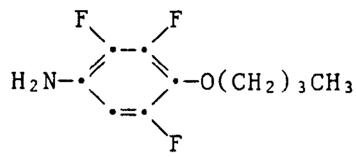
,

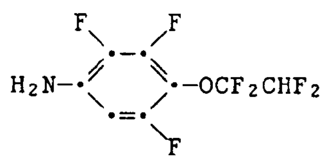

und

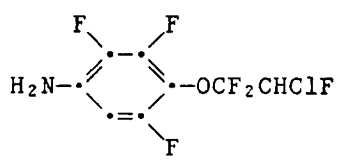
.

**13.** Die Verbindung der Formel

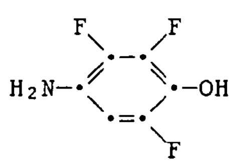
.

**14.** Verbindungen der Formel IX

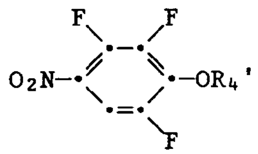 (IX),

worin $R_4$' für $C_2$-$C_{10}$-Alkyl steht.

**15.** Die Verbindung gemäss Anspruch 14 der Formel

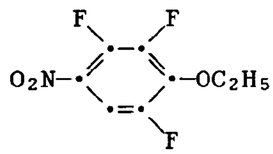 ,

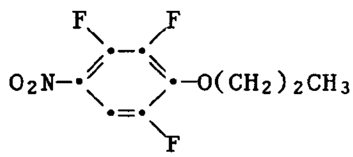 ,

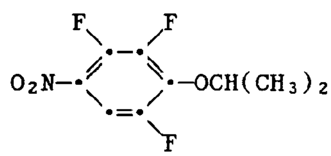 ,

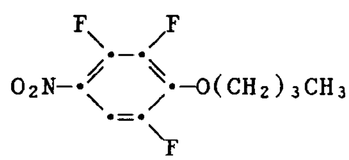

und

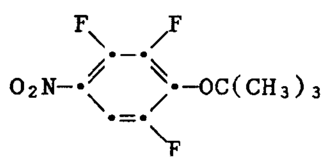 .

**Patentansprüche für folgenden Vertragsstaat : ES**

**1.** Verfahren zur Herstellung einer Verbindung der Formel I

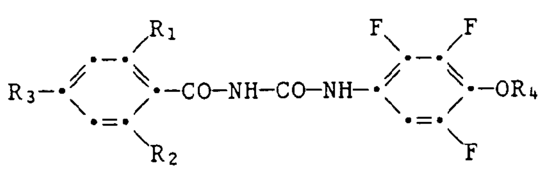 (I),

worin $R_1$ für Wasserstoff, Fluor oder Chlor; $R_2$ für Fluor oder Chlor; $R_3$ für Wasserstoff oder Fluor; und $R_4$ für $C_1$-$C_{10}$-Alkyl, -C(X)F-$C_1$-$C_7$-Alkyl oder ein- oder mehrfach durch Halogen substituiertes -C(X)F-$C_1$-$C_7$-Alkyl stehen, wobei X Wasserstoff oder Halogen bedeutet, dadurch gekennzeichnet, dass man

a) ein Anilin der Formel II

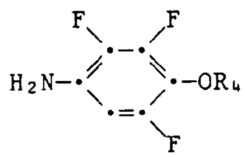

(II)

mit einem Benzoylisocyanat der Formel III

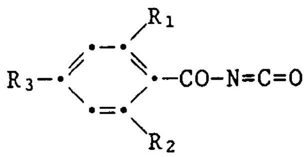

(III),

oder

b) ein Isocyanat der Formel IV

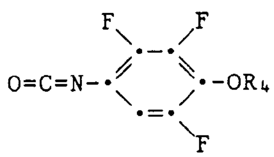

(IV)

mit einem Benzamid der Formel V

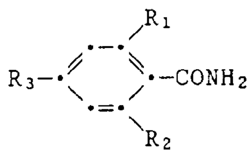

(V),

oder

c) ein Anilin der Formel II mit einem Urethan der Formel VI

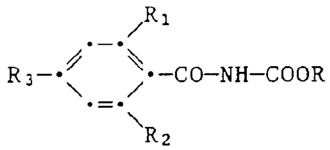

(VI)

umsetzt, wobei in den Formeln II, III, IV, V und VI $R_1$, $R_2$, $R_3$ und $R_4$ die angegebene Bedeutung haben und in Formel VI R für einen gegebenenfalls halogenierten $C_1$-$C_8$-Alkylrest steht.

2. Verfahren zur Herstellung von Verbindungen der Formel I gemäss Anspruch 1, worin $R_1$ für Wasserstoff oder Fluor; $R_2$ für Fluor oder Chlor; $R_3$ für Wasserstoff oder Fluor; und $R_4$ für $C_1$-$C_7$-Alkyl, -C(X)F-$C_1$-$C_5$-Alkyl oder ein- oder mehrfach durch Halogen substituiertes -C(X)F-$C_1$-$C_5$-Alkyl stehen, wobei X Wasserstoff, Fluor oder Chlor bedeutet.

3. Verfahren zur Herstellung von Verbindungen der Formel I gemäss Anspruch 2, worin $R_1$ für Wasserstoff oder Fluor; $R_2$ für Fluor oder Chlor; $R_3$ für Wasserstoff oder Fluor; und $R_4$ für $C_1$-$C_3$-Alkyl, -$CF_2$-$C_1$-$C_3$-Alkyl

oder ein- oder mehrfach durch Fluor substituiertes $-CF_2-C_1-C_3$-Alkyl stehen.

4. Verfahren zur Herstellung von Verbindungen gemäss einem der Ansprüche 1 bis 3 der Formeln

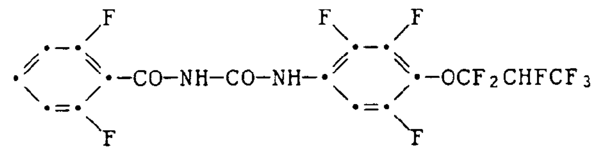 ,

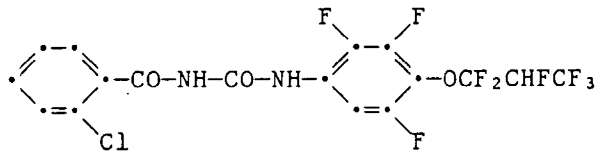 ,

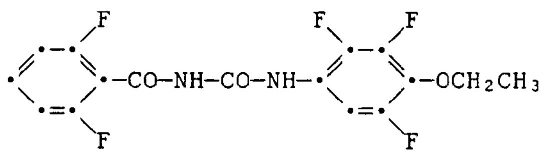 ,

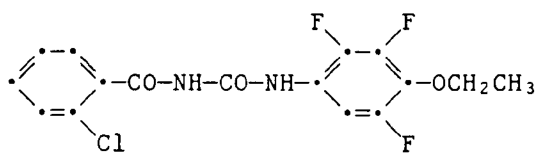 ,

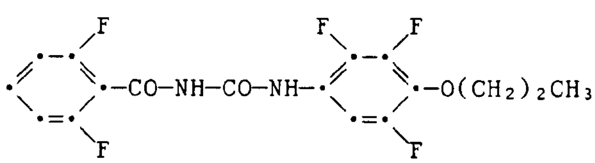 ,

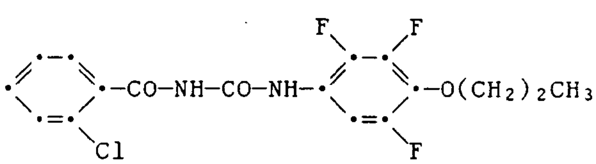 ,

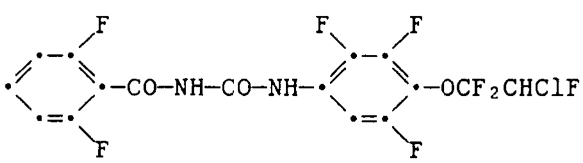 ,

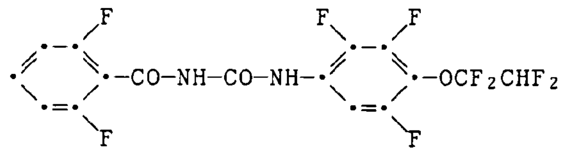 ,

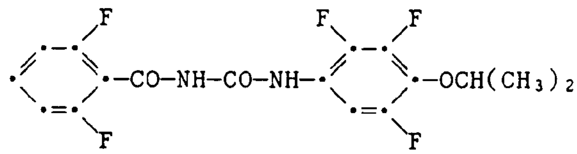 ,

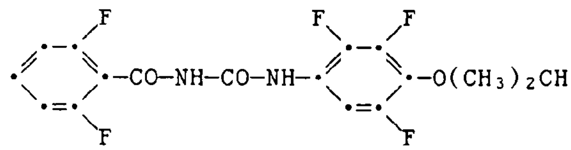 ,

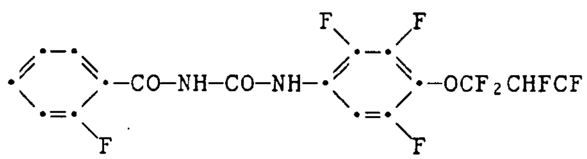 ,

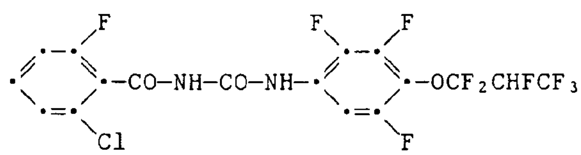 ,

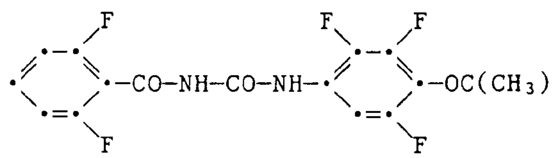 ,

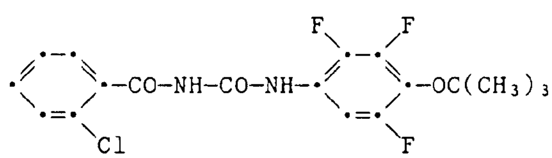 ,

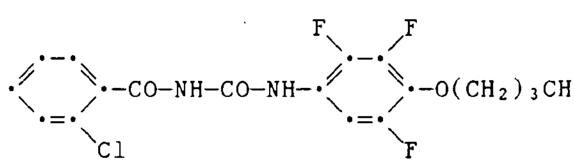 ,

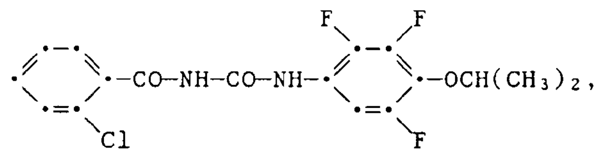

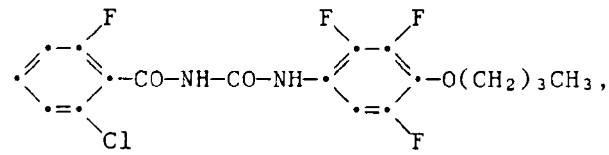

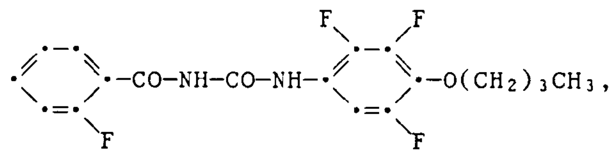

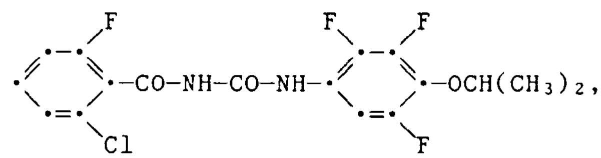

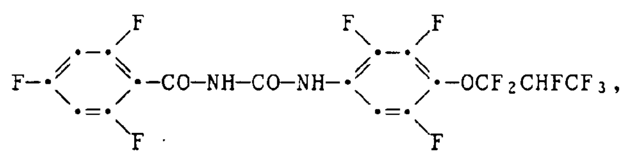

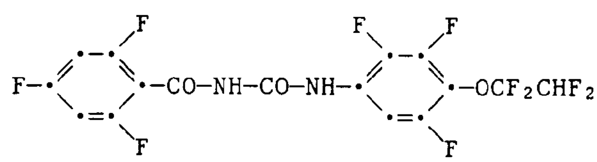

und

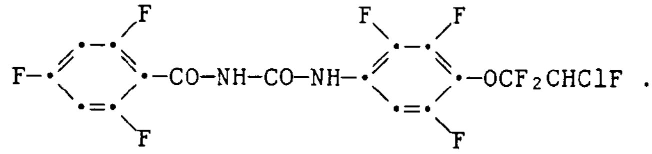

5. Schädlingsbekämpfungsmittel, welches als aktive Komponente eine wirksame Menge einer Verbindung, erhältlich gemäss Anspruch 1, der Formel I

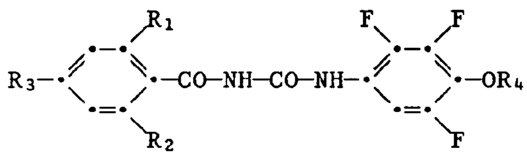

(I),

worin $R_1$ für Wasserstoff, Fluor oder Chlor; $R_2$ für Fluor oder Chlor; $R_3$ für Wasserstoff oder Fluor; und $R_4$ für $C_1$-$C_{10}$-Alkyl, -C(X)F-$C_1$-$C_7$-Alkyl oder ein- oder mehrfach durch Halogen substituiertes -C(X)F-$C_1$-$C_7$-Alkyl stehen, wobei X Wasserstoff oder Halogen bedeutet, zusammen mit geeigneten Trägern und/oder Zuschlagstoffen enthält.

6. Verfahren zur Herstellung eines Mittels gemäss Anspruch 5, dadurch gekennzeichnet, dass man den Wirkstoff mit dem (den) Hilfsstoff(en) innig vermischt.

7. Verwendung einer Verbindung, erhältlich gemäss Anspruch 1, der Formel I

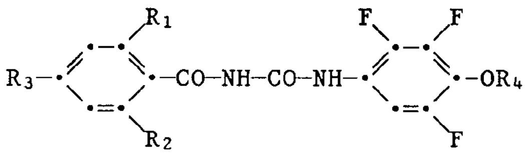

(I),

worin $R_1$ für Wasserstoff, Fluor oder Chlor; $R_2$ für Fluor oder Chlor; $R_3$ für Wasserstoff oder Fluor; und $R_4$ für $C_1$-$C_{10}$-Alkyl, -C(X)F-$C_1$-$C_7$-Alkyl oder ein- oder mehrfach durch Halogen substituiertes -C(X)F-$C_1$-$C_7$-Alkyl stehen, wobei X Wasserstoff oder Halogen bedeutet, zur Kontrolle von Schädlingen an Tieren und Pflanzen.

8. Verwendung gemäss Anspruch 7 einer Verbindung der Formel I zur Kontrolle von Insekten und Arachniden.

9. Verwendung gemäss Anspruch 8 einer Verbindung der Formel I zur Kontrolle larvaler Stadien von pflanzenschädigenden Insekten.

10. Verfahren zur Kontrolle von Schädlingen an Tieren und Pflanzen, dadurch gekennzeichnet, dass man die Schädlinge in ihren verschiedenen Entwicklungsstadien mit einer verbindung, erhältlich gemäss Anspruch 1, der Formel I

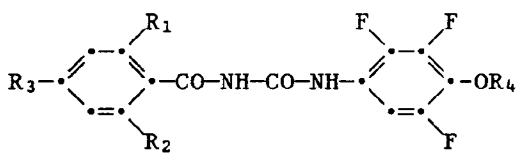

(I),

worin $R_1$ für Wasserstoff, Fluor oder Chlor; $R_2$ für Fluor oder Chlor; $R_3$ für Wasserstoff oder Fluor; und $R_4$ für $C_1$-$C_{10}$-Alkyl, -C(X)F-$C_1$-$C_7$-Alkyl oder ein- oder mehrfach durch Halogen substituiertes -C(X)F-$C_1$-$C_7$-Alkyl stehen, wobei X Wasserstoff oder Halogen bedeutet, in Kontakt bringt.

11. Verfahren zur Herstellung einer Verbindung der Formel II

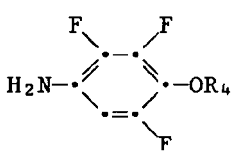

(II),

worin $R_4$ für -$CF_2$-CHF-$C_1$-$C_6$-Alkyl, ein- oder mehrfach durch Halogen substituiertes -$CF_2$-CHF-$C_1$-$C_6$-Alkyl, -$CF_2$-$CHF_2$ oder -$CF_2$-CHFCl steht, dadurch gekennzeichnet, dass man eine Verbindung der Formel

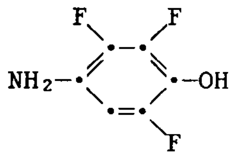

(VII)

mit $CF_2$=$CF_2$, $CF_2$=CFCl, $CF_2$=CF-$C_1$-$C_6$-Alkyl oder einem ein- oder mehrfach durch Halogen substituierten $CF_2$=CF-$C_1$-$C_6$-Alkyl umsetzt.

**12.** Verfahren gemäss Anspruch 11 zur Herstellung von Verbindungen der Formeln

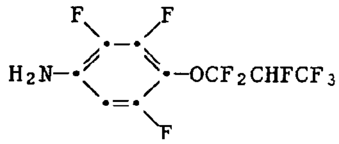

,

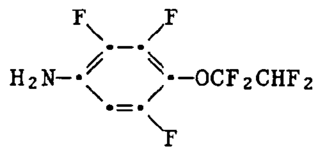

und

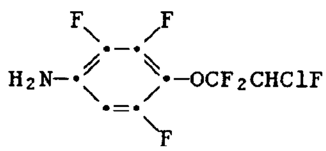

.

**13.** Verfahren zur Herstellung der Verbindung der Formel

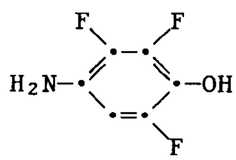

,

dadurch gekennzeichnet, dass man eine Verbindung der Formel

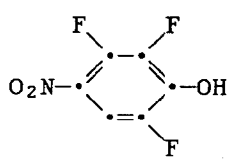

(VIII)

katalytisch hydriert oder chemisch reduziert.

**14.** Verfahren zur Herstellung einer Verbindung der Formel IX

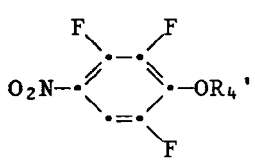

(IX),

worin $R_4'$ für $C_2$-$C_{10}$-Alkyl steht, dadurch gekennzeichnet, dass man 2,3,4,5-Tetrafluorintrobenzol.

**15.** Verfahren gemäss Anspruch 14 zur Herstellung von Verbindungen der Formeln

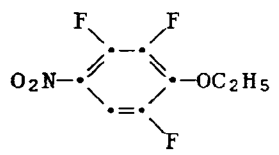

,

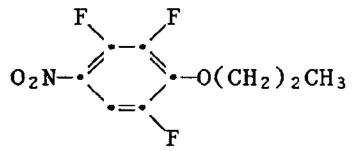

,

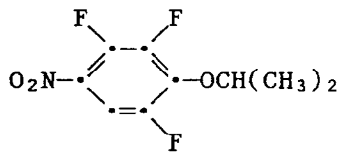

,

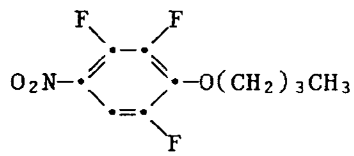

und

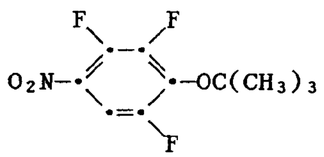

.

mit einem Metallalkoholat der Formel $MetOR_4'$, worin Met für ein Erdalkali- oder vorzugsweise ein Alkalimetall steht und $R_4'$ die oben angegebene Bedeutung hat, umsetzt.

## Claims

### Claims for the following Contracting States : AT, BE, CH, DE, FR, GB, GR, IT, LI, NL

**1.** A compound of formula I

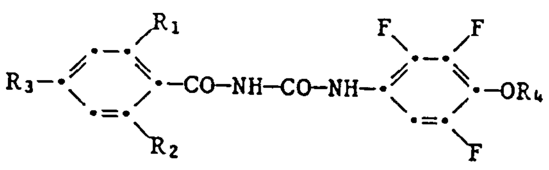

(I)

in which $R_1$ is hydrogen, fluorine or chlorine; $R_2$ is fluorine or chlorine; $R_3$ is hydrogen or fluorine; and $R_4$ is $C_1$-$C_{10}$alkyl, -C(X)F-$C_1$-$C_7$alkyl, or -C(X)F-$C_1$-$C_7$alkyl that is mono- or poly-substituted by halogen, X being hydrogen or halogen.

2. A compound of formula I according to claim 1, in which $R_1$ is hydrogen or fluorine; $R_2$ is fluorine or chlorine; $R_3$ is hydrogen or fluorine; and $R_4$ is $C_1$-$C_7$alkyl, -C(X)F-$C_1$-$C_5$alkyl, or -C(X)F-$C_1$-$C_5$alkyl that is mono- or poly-substituted by halogen, X being hydrogen, fluorine or chlorine.

3. A compound of formula I according to claim 2, in which $R_1$ is hydrogen or fluorine; $R_2$ is fluorine or chlorine; $R_3$ is hydrogen or fluorine; and $R_4$ is $C_1$-$C_3$alkyl, -$CF_2$-$C_1$-$C_3$alkyl, or -$CF_2$-$C_1$-$C_3$alkyl that is mono- or poly-substituted by fluorine.

4. A compound according to any one of claims 1 to 3 of the formula

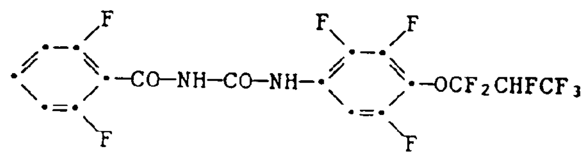 ,

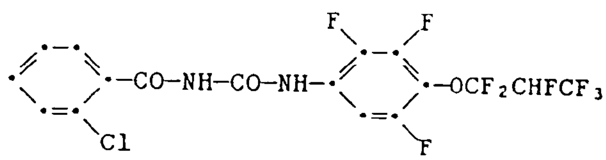 ,

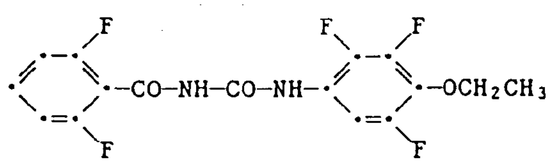 ,

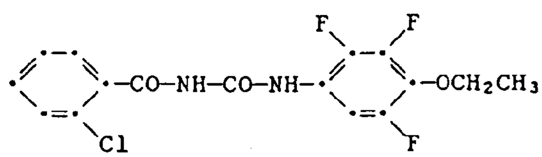 ,

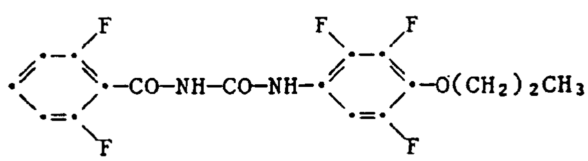 ,

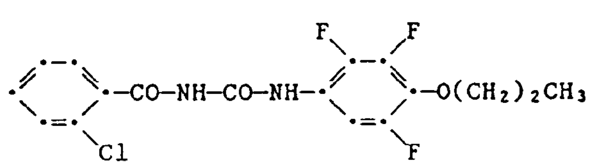 ,

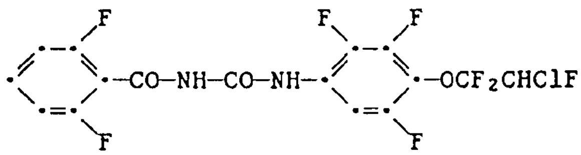
,

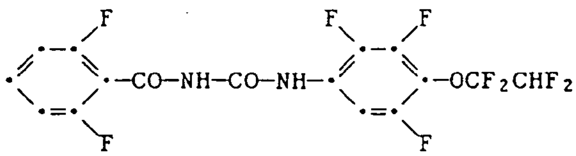
,

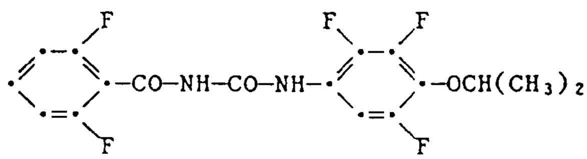
,

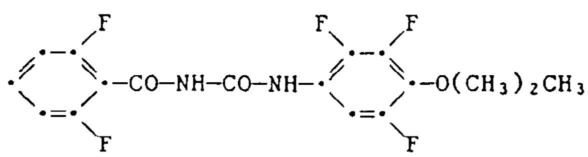
,

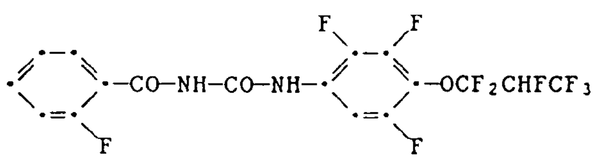
,

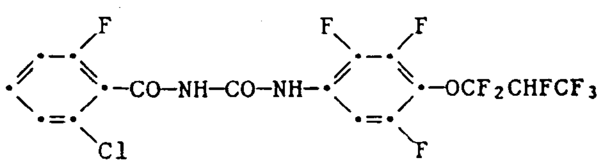
,

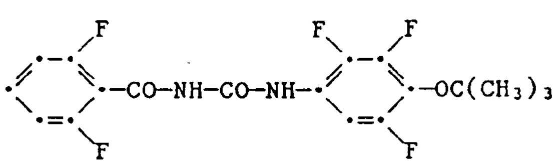
,

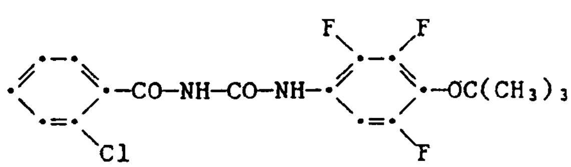
,

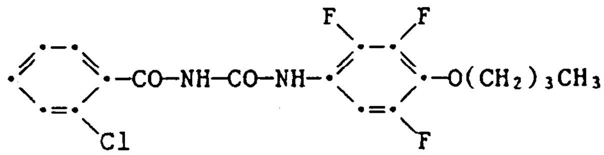
,

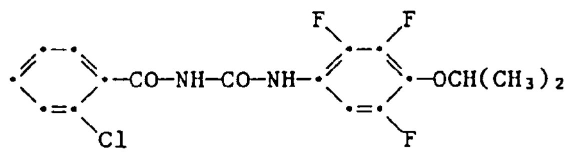
,

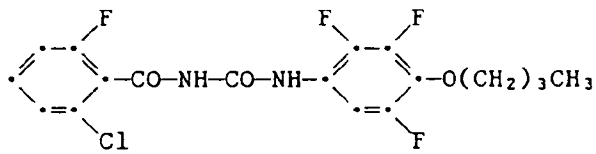
,

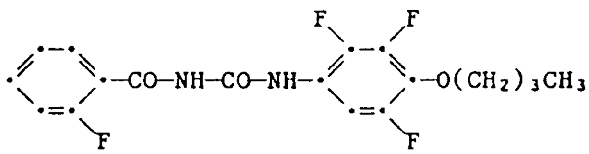
,

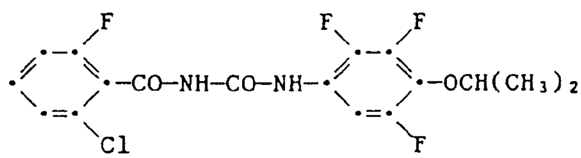
,

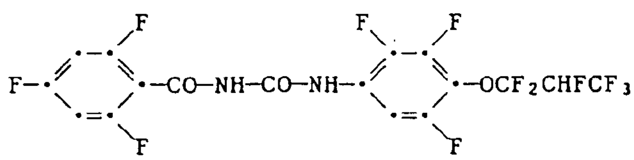
,

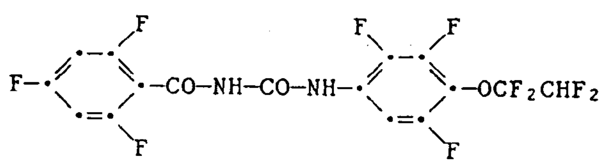

or

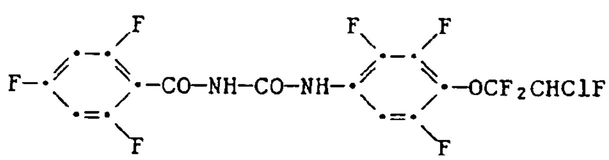
.

5. A process for the preparation of a compound of formula I

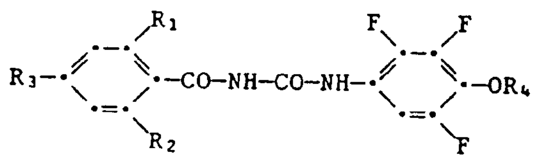

(I),

in which $R_1$ is hydrogen, fluorine or chlorine; $R_2$ is fluorine or chlorine; $R_3$ is hydrogen or fluorine; and $R_4$ is $C_1$-$C_{10}$alkyl, -C(X)F-$C_1$-$C_7$alkyl, or -C(X)F-$C_1$-$C_7$alkyl that is mono- or poly-substituted by halogen, X being hydrogen or halogen, which comprises

a) reacting an aniline of formula II

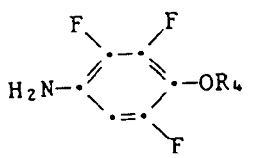

(II)

with a benzoyl isocyanate of formula III

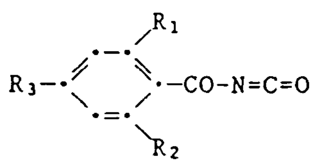

(III)

or

b) reacting an isocyanate of formula IV

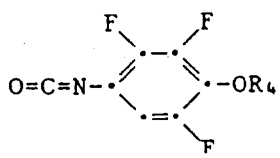

(IV)

with a benzamide of formula V

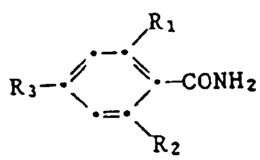

(V)

or

c) reacting an aniline of formula II with a urethane of formula VI

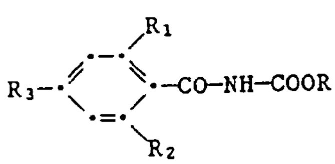

(VI),

$R_1$, $R_2$, $R_3$ and $R_4$ in formulae II, III, IV, V and VI having the meanings given and R in formula VI being an unsubstituted or halogenated $C_1$-$C_8$alkyl radical.

6. A pesticidal composition comprising as active component an effective amount of a compound of formula I

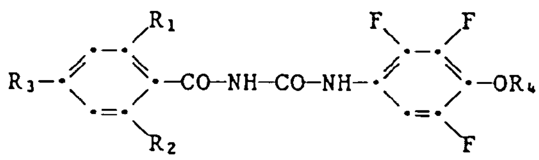

(I)

in which $R_1$ is hydrogen, fluorine or chlorine; $R_2$ is fluorine or chlorine; $R_3$ is hydrogen or fluorine; and $R_4$ is $C_1$-$C_{10}$alkyl, -C(X)F-$C_1$-$C_7$alkyl, or -C(X)F-$C_1$-$C_7$alkyl that is mono- or poly-substituted by halogen, X being hydrogen or halogen, together with suitable carriers and/or adjuvants.

7. The use of a compound of formula I

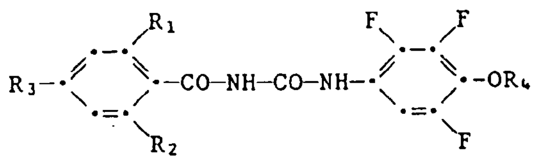

(I)

in which $R_1$ is hydrogen, fluorine or chlorine; $R_2$ is fluorine or chlorine; $R_3$ is hydrogen or fluorine; and $R_4$ is $C_1$-$C_{10}$alkyl, -C(X)F-$C_1$-$C_7$alkyl, or -C(X)F-$C_1$-$C_7$alkyl that is mono- or poly-substituted by halogen, X being hydrogen or halogen, for controlling pests in and on animals and plants.

8. Use according to claim 7 of a compound of formula I for controlling insects and arachnids.

9. Use according to claim 8 of a compound of formula I for controlling larval stages of plant-destructive insects.

10. A method of controlling pests in and on animals and plants, which comprises bringing the pests, in their various stages of development, into contact with a compound of formula I

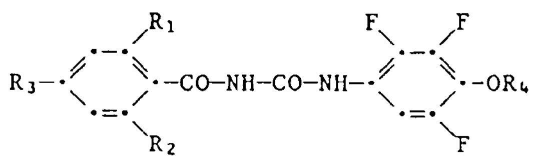

(I)

in which $R_1$ is hydrogen, fluorine or chlorine; $R_2$ is fluorine or chlorine; $R_3$ is hydrogen or fluorine; and $R_4$ is $C_1$-$C_{10}$alkyl, -C(X)F-$C_1$-$C_7$alkyl, or -C(X)F-$C_1$-$C_7$alkyl that is mono- or poly-substituted by halogen, X being hydrogen or halogen.

11. A compound of formula II

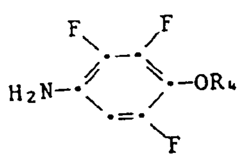

(II)

in which $R_4$ is $C_1$-$C_{10}$alkyl, -C(X)F-$C_1$-$C_7$alkyl, or -C(X)F-$C_1$-$C_7$alkyl that is mono- or poly-substituted by halogen, X being hydrogen or halogen.

12. A compound according to claim 11 of formula

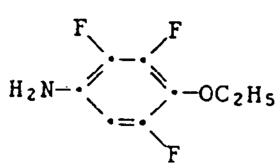

,

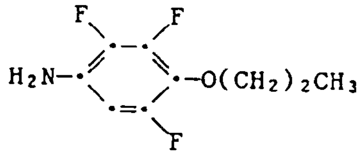

,

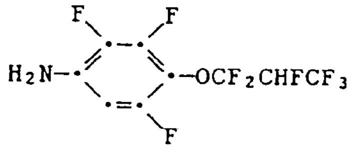

,

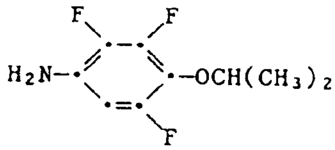

,

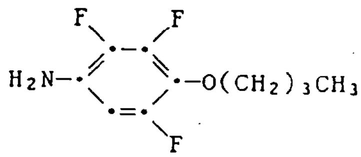

,

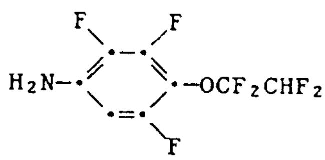

or

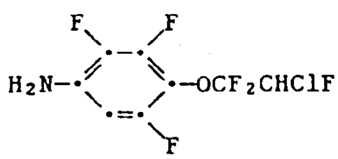

.

**13.** The compound of formula

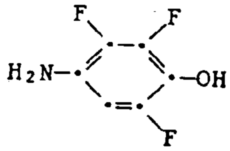

.

**14.** A compound of formula IX

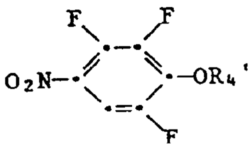

(IX)

in which $R_4$' is $C_2$-$C_{10}$alkyl.

**15.** A compound according to claim 14 of formula

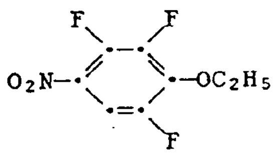

,

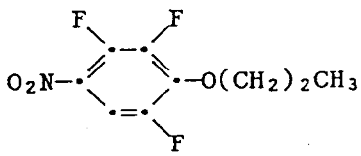

,

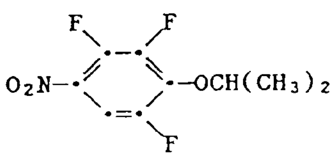

,

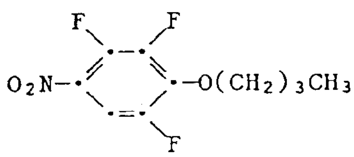

or

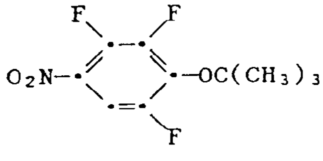

.

**Claims for the following Contracting State : ES**

**1.** A process for the preparation of a compound of formula I

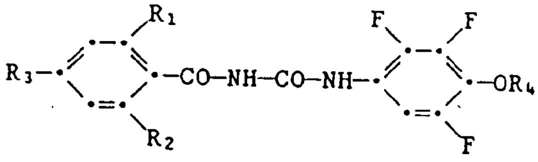

(I)

in which $R_1$ is hydrogen, fluorine or chlorine; $R_2$ is fluorine or chlorine; $R_3$ is hydrogen or fluorine; and $R_4$

is $C_1$-$C_{10}$alkyl, -C(X)F-$C_1$-$C_7$alkyl, or -C(X)F-$C_1$-$C_7$alkyl that is mono- or poly-substituted by halogen, X being hydrogen or halogen, which comprises

a) reacting an aniline of formula II

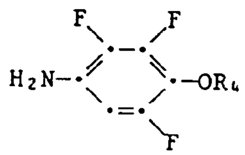

(II)

with a benzoyl isocyanate of formula III

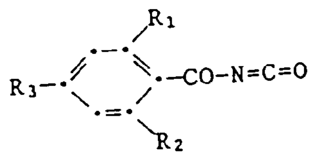

(III)

or

b) reacting an isocyanate of formula IV

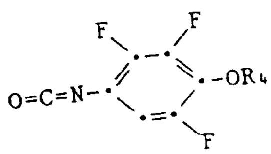

(IV)

with a benzamide of formula V

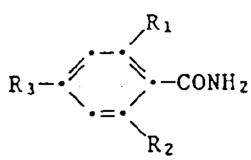

(V)

or

c) reacting an aniline of formula II with a urethane of formula VI

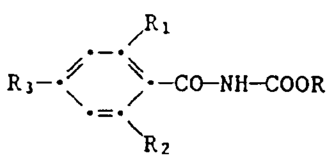

(VI),

$R_1$, $R_2$, $R_3$ and $R_4$ in formulae II, III, IV, V and VI having the meanings given and R in formula VI being an unsubstituted or halogenated $C_1$-$C_8$alkyl radical.

2. A process for the preparation of a compound of formula I according to claim 1, in which $R_1$ is hydrogen or fluorine; $R_2$ is fluorine or chlorine; $R_3$ is hydrogen or fluorine; and $R_4$ is $C_1$-$C_7$alkyl, -C(X)F-$C_1$-$C_5$alkyl, or -C(X)F-$C_1$-$C_5$alkyl that is mono- or poly-substituted by halogen, X being hydrogen, fluorine or chlorine.

3. A process for the preparation of a compound of formula I according to claim 2, in which $R_1$ is hydrogen or fluorine; $R_2$ is fluorine or chlorine; $R_3$ is hydrogen or fluorine; and $R_4$ is $C_1$-$C_3$alkyl, -$CF_2$-$C_1$-$C_3$alkyl, or -$CF_2$-$C_1$-$C_3$alkyl that is mono- or poly-substituted by fluorine.

4. A process for the preparation of a compound according to any one of claims 1 to 3 of the formula

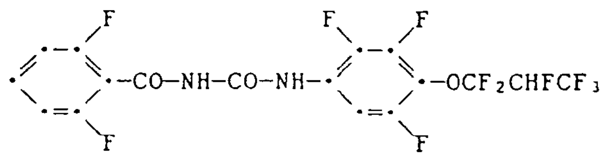 ,

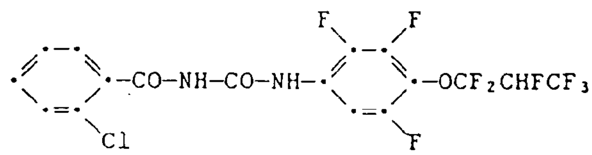 ,

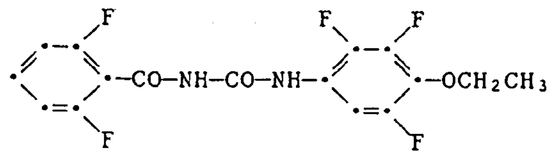 ,

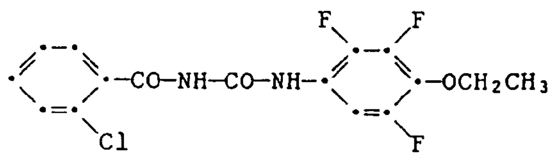 ,

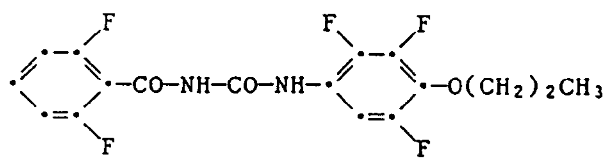 ,

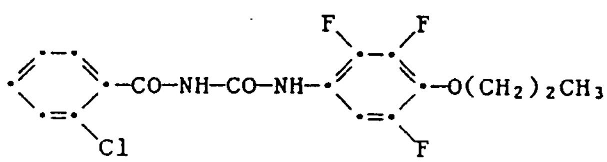 ,

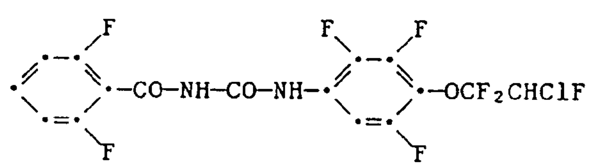 ,

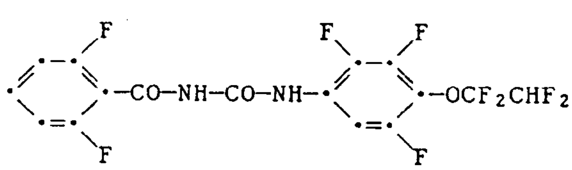 ,

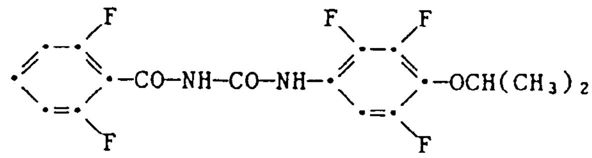,

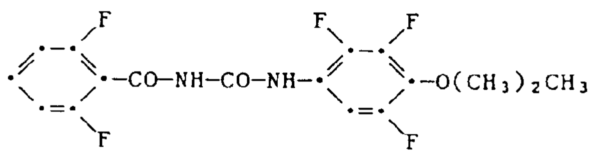,

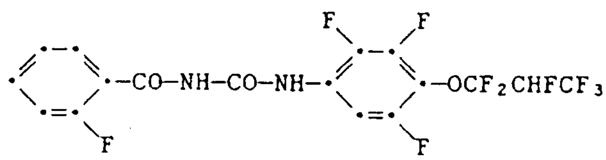,

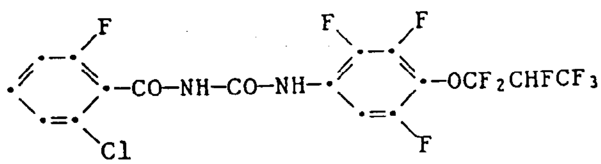,

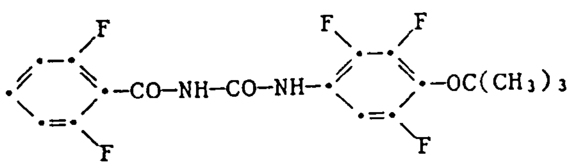,

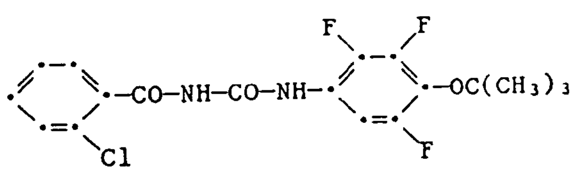,

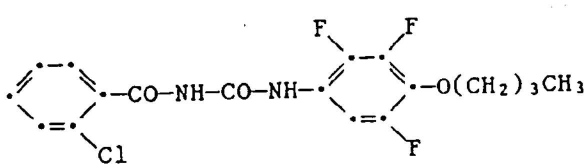,

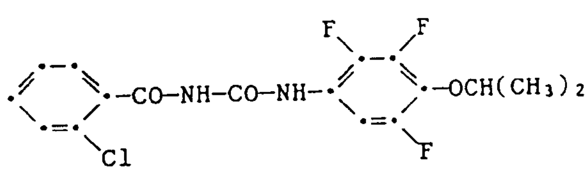,

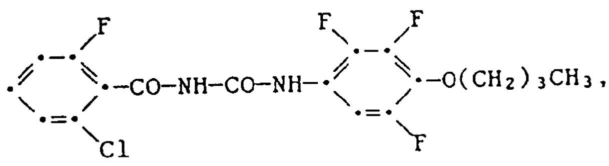

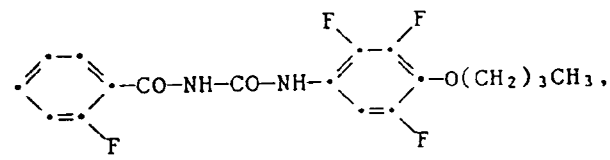

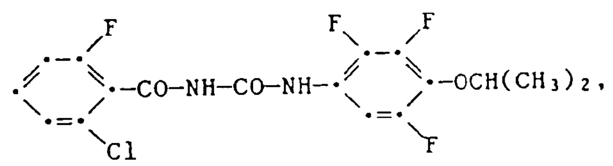

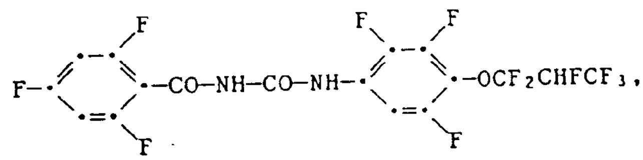

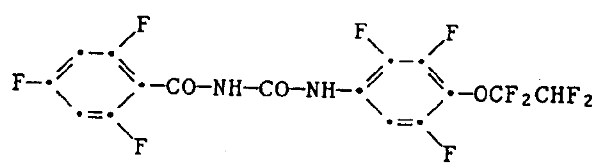

or

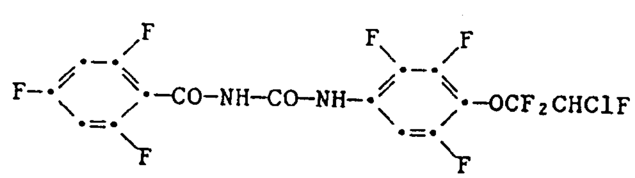

.

5. A pesticidal composition comprising as active component an effective amount of a compound, obtainable according to claim 1, of formula I

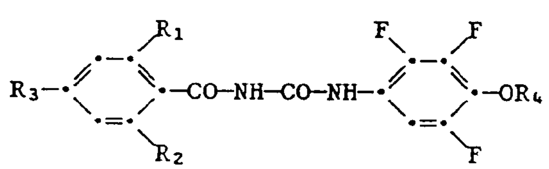
(I)

in which $R_1$ is hydrogen, fluorine or chlorine; $R_2$ is fluorine or chlorine; $R_3$ is hydrogen or fluorine; and $R_4$ is $C_1$-$C_{10}$alkyl, -C(X)F-$C_1$-$C_7$alkyl, or -C(X)F-$C_1$-$C_7$alkyl that is mono- or poly-substituted by halogen, X being hydrogen or halogen, together with suitable carriers and/or adjuvants.

6. A process for the preparation of a composition according to claim 5, which comprises mixing the active

ingredient homogeneously with the adjuvant(s).

7. The use of a compound, obtainable according to claim 1, of formula I

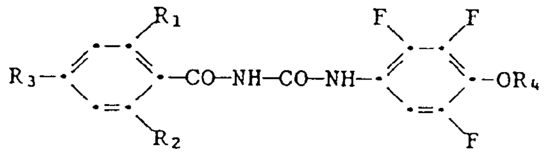

(I)

in which $R_1$ is hydrogen, fluorine or chlorine; $R_2$ is fluorine or chlorine; $R_3$ is hydrogen or fluorine; and $R_4$ is $C_1$-$C_{10}$alkyl, -C(X)F-$C_1$-$C_7$alkyl, or -C(X)F-$C_1$-$C_7$alkyl that is mono- or poly-substituted by halogen, X being hydrogen or halogen, for controlling pests in and on animals and plants.

8. Use according to claim 7 of a compound of formula I for controlling insects and arachnids.

9. Use according to claim 8 of a compound of formula I for controlling larval stages of plant-destructive insects.

10. A method of controlling pests in and on animals and plants, which comprises bringing the pests, in their various stages of development, into contact with a compound, obtainable according to claim 1, of formula I

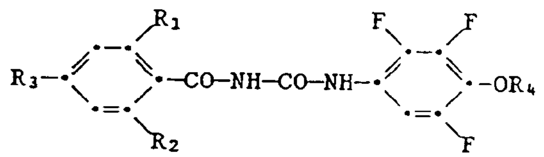

(I)

in which $R_1$ is hydrogen, fluorine or chlorine; $R_2$ is fluorine or chlorine; $R_3$ is hydrogen or fluorine; and $R_4$ is $C_1$-$C_{10}$alkyl, -C(X)F-$C_1$-$C_7$alkyl, or -C(X)F-$C_1$-$C_7$alkyl that is mono- or poly-substituted by halogen, X being hydrogen or halogen.

11. A process for the preparation of a compound of formula II

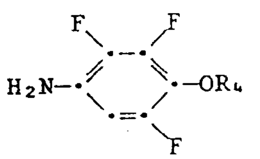

(II)

in which $R_4$ is $-CF_2$-CHF-$C_1$-$C_6$alkyl, $-CF_2$-CHF-$C_1$-$C_6$alkyl that is mono- or poly-substituted by halogen, $-CF_2-CHF_2$ or $-CF_2$-CHFCl, which comprises reacting a compound of formula

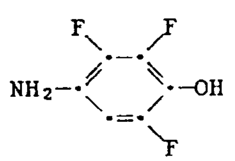

(VII)

with $CF_2=CF_2$, $CF_2CFCl$, $CF_2=CF$-$C_1$-$C_6$alkyl, or $CF_2=CF$-$C_1$-$C_6$alkyl that is mono- or poly-substituted by halogen.

12. A process according to claim 11 for the preparation of a compound of the formula

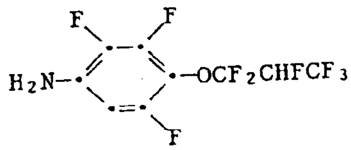

,

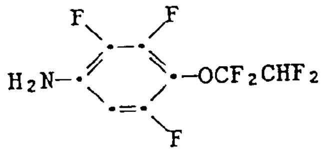

or

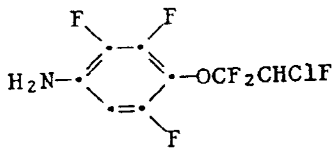

.

13. A process for the preparation of the compound of formula

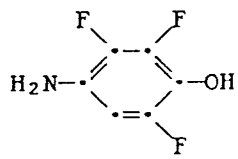

which comprises catalytically hydrogenating or chemically reducing a compound of formula

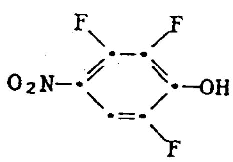

(VIII).

14. A process for the preparation of a compound of formula IX

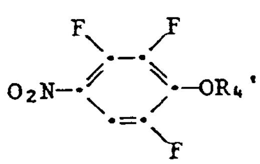

(IX)

in which $R_4$' is $C_2$-$C_{10}$alkyl, which comprises reacting 2,3,4,5-tetrafluoronitrobenzene with a metal alcoholate of formula $MetOR_4$' in which Met is an alkaline earth metal or, preferably, an alkali metal, and $R_4$' has the meaning given above.

15. A process according to claim 14 for the preparation of a compound of formula

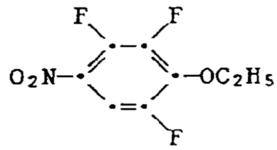 ,

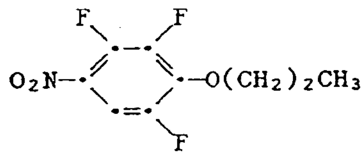 ,

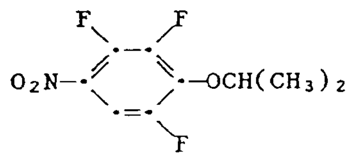 ,

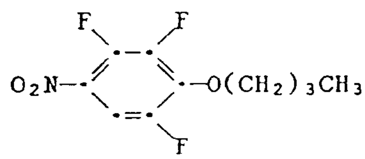

or

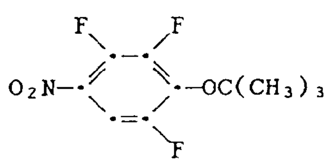 .

## Revendications

**Revendications pour les Etats contractants suivants : AT, BE, CH, DE, FR, GB, GR, IT, LI, NL**

**1.** Composés de formule I

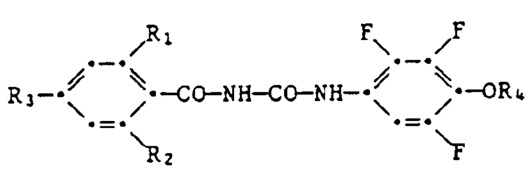 (I),

dans laquelle $R_1$ représente l'hydrogène ou le fluor ou le chlore; $R_2$ le fluor ou le chlore; $R_3$ l'hydrogène ou le fluor; et $R_4$ représente un groupe alkyle en C1-C10, -C(X)F-alkyle en C1-C7 ou un groupe -C(X)F-alkyle en C1-C7 substitué par un ou plusieurs atomes d'halogènes, X représentant l'hydrogène ou un halogène.

**2.** Composés de formule I de la revendication 1, dans laquelle $R_1$ représente l'hydrogène ou le fluor; $R_2$ le

fluor ou le chlore; $R_3$ l'hydrogène ou le fluor; et $R_4$ un groupe alkyle en C1-C7, -C(X)F-alkyle en C1-C5 ou -C(X)F-alkyle en C1-C5 substitué par un ou plusieurs atomes d'halogènes; X représentant l'hydrogène, le fluor ou le chlore.

**3.** Composés de formule I de la revendication 2, dans laquelle $R_1$ représente l'hydrogène ou le fluor; $R_2$ le fluor ou le chlore; $R_3$ l'hydrogène ou le fluor; et $R_4$ un groupe alkyle en C1-C3, $-CF_2$-alkyle en C1-C3 ou $-CF_2$-alkyle en C1-C3 substitué par un ou plusieurs atomes de fluor.

**4.** Les composés selon une des revendications 1 à 3, de formules

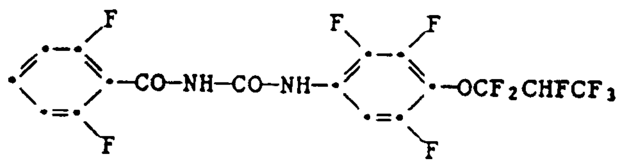
,

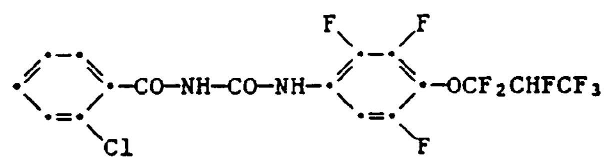
,

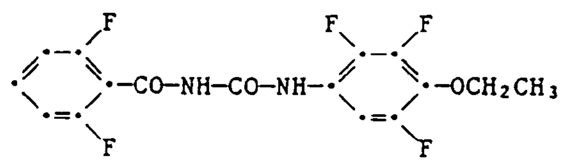
,

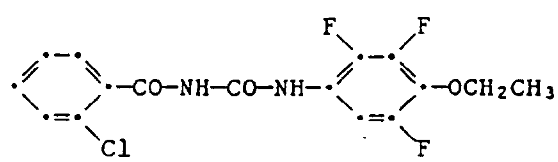
,

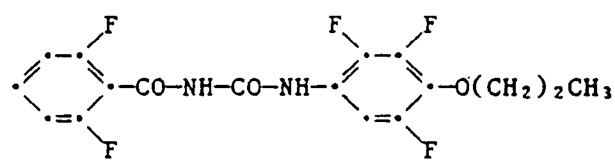
,

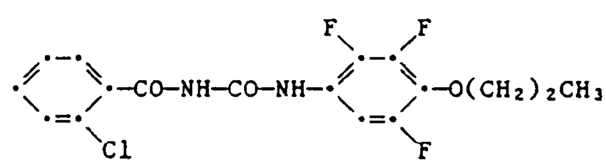
,

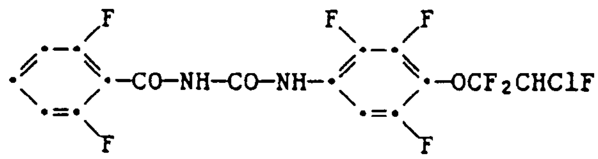 ,

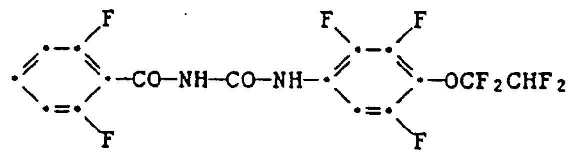 ,

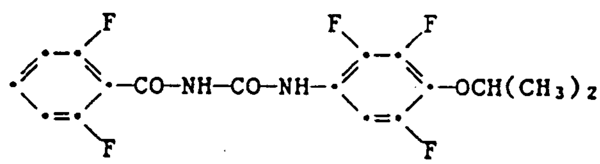 ,

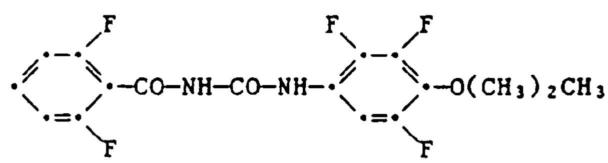 ,

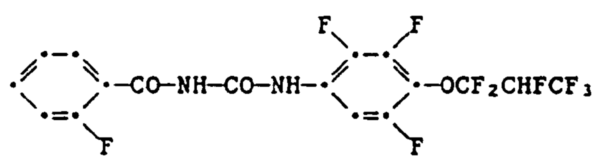 ,

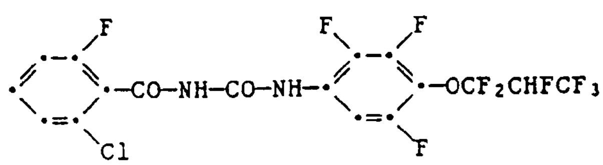 ,

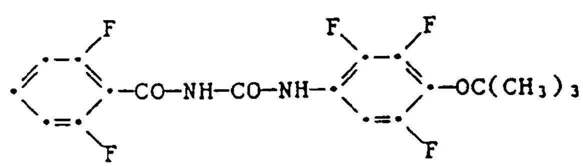 ,

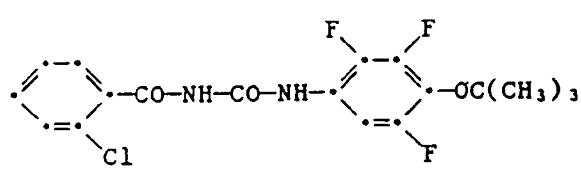 ,

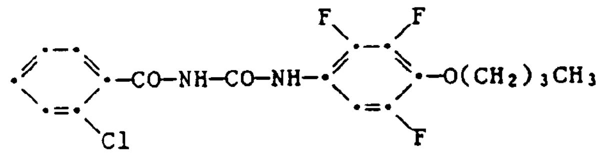,

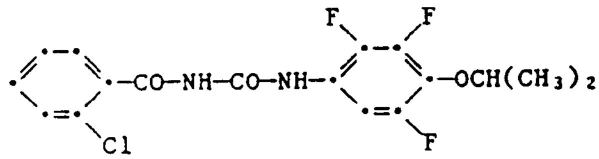,

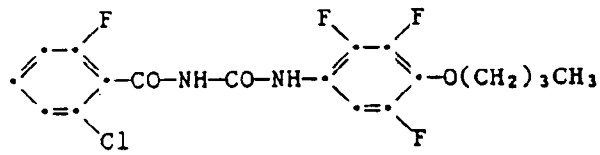,

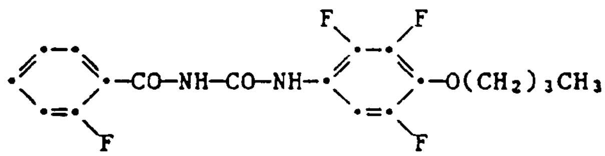,

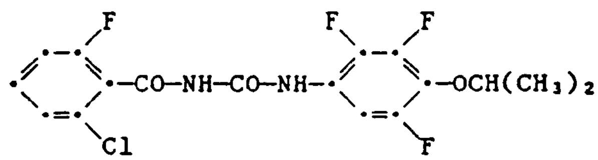,

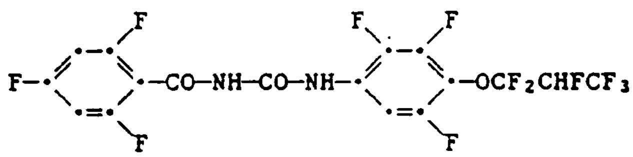,

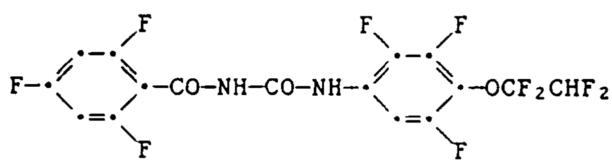

et

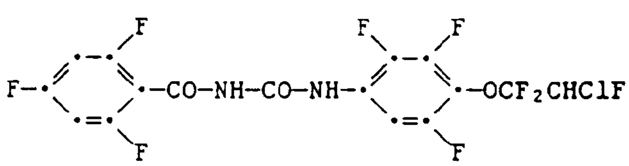 .

**5.** Procédé de préparation d'un composé de formule I

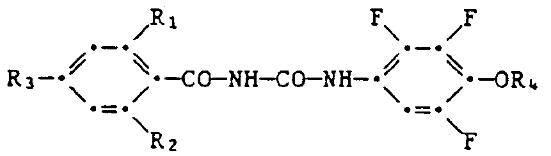

(I),

dans laquelle $R_1$ représente l'hydrogène, le fluor ou le chlore; $R_2$ le fluor ou le chlore; $R_3$ l'hydrogène ou le fluor; et $R_4$ un groupe alkyle en C1-C10, -C(X)F-alkyle en C1-C7 ou -C(X)F-alkyle en C1-C7 substitué par un ou plusieurs atomes d'halogènes; X représentant l'hydrogène ou un halogène, caractérisé en ce que

a) on fait réagir une aniline de formule II

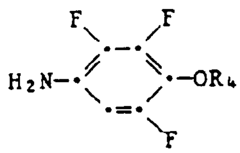

(II)

avec un isocyanate de benzoyle de formule III

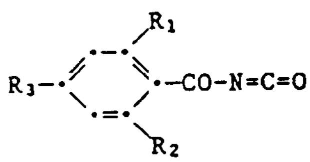

(III)

ou bien

b) on fait réagir un isocyanate de formule IV

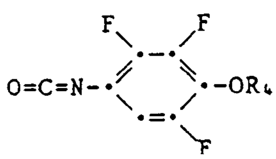

(IV)

avec un benzamide de formule V

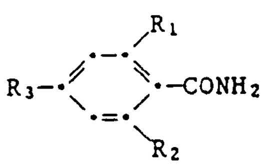

(V),

ou bien

c) on fait réagir une aniline de formule II avec un uréthanne de formule VI

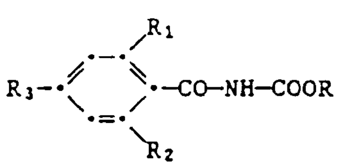

(VI)

les symboles $R_1$, $R_2$, $R_3$ et $R_4$ ayant, dans les formules II, III, IV, V et VI, les significations indiquées ci-dessus, et le symbole R de la formule VI représentant un groupe alkyle en C1-C8 éventuellement halogéné.

6. Produit parasiticide contenant en tant que composant actif, en quantité efficace, un composé de formule I

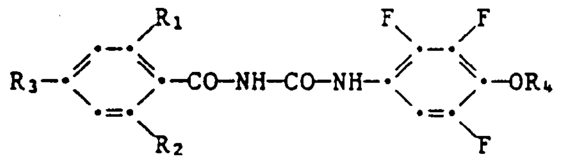

(I),

dans laquelle $R_1$ représente l'hydrogène, le fluor ou le chlore; $R_2$ représente le fluor ou le chlore; $R_3$ l'hydrogène ou le fluor; et $R_4$ un groupe alkyle en C1-C10, -C(X)F-alkyle en C1-C7 ou -C(X)F-alkyle en C1-C7 substitué par un ou plusieurs atomes d'halogènes, X représentant l'hydrogène ou un halogène, avec des véhicules et/ou additifs appropriés.

7. Utilisation d'un composé de formule I

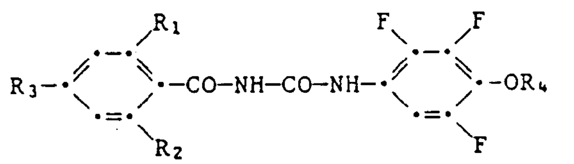

(I),

dans laquelle $R_1$ représente l'hydrogène, le fluor ou le chlore; $R_2$ le fluor ou le chlore; $R_3$ l'hydrogène ou le fluor; et $R_4$ un groupe alkyle en C1-C10, -C(X)F-alkyle en C1-C7 ou -C(X)F-alkyle en C1-C7 substitué par un ou plusieurs atomes d'halogènes; X représentant l'hydrogène ou un halogène, pour la lutte contre les parasites des animaux et des végétaux.

8. Utilisation selon la revendication 7 d'un composé de formule I pour la lutte contre les insectes et les arachnides.

9. Utilisation selon la revendication 8 d'un composé de formule I pour la lutte contre les stades larvaires des insectes nuisibles pour les végétaux.

10. Procédé pour combattre les parasites des animaux et des végétaux, caractérisé en ce que l'on met les parasites, dans leurs divers stades de développement, en contact avec un composé de formule I

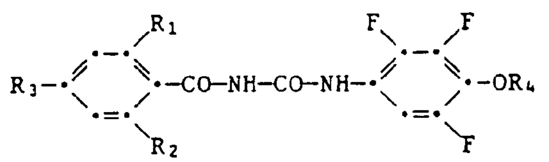

(I),

dans laquelle $R_1$ représente l'hydrogène, le fluor ou le chlore; $R_2$ représente le fluor ou le chlore; $R_3$ représente l'hydrogène ou le fluor; et $R_4$ représente un groupe alkyle en C1-C10, -C(X)F-alkyle en C1-C7 ou -C(X)F-alkyle en C1-C7 substitué par un ou plusieurs atomes d'halogènes, X représentant l'hydrogène ou un halogène.

11. Composés de formule II

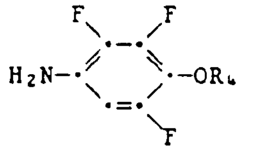

(II),

dans laquelle $R_4$ représente un groupe alkyle en C1-C10, -C(X)F-alkyle en C1-C7 ou -C(X)F-alkyle en C1-C7 substitué par un ou plusieurs atomes d'halogènes, X représentant l'hydrogène ou un halogène.

12. Les composés selon revendication 11, de formules

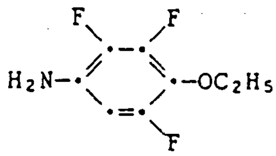

,

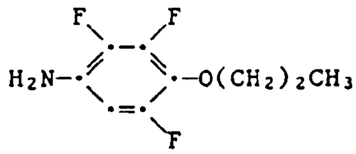

,

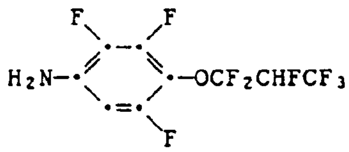

,

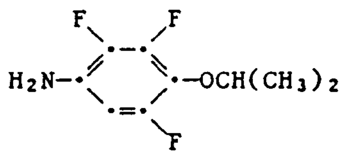

,

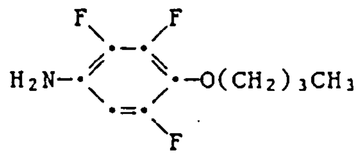

,

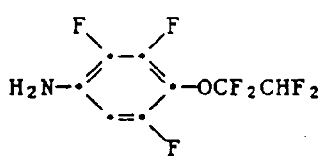

et

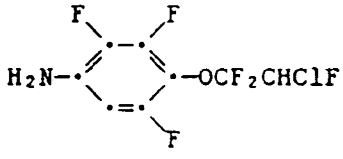

.

13. Le composé de formule

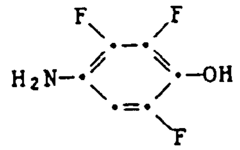

**14.** Composés de formule IX

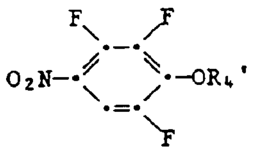

(IX),

dans laquelle $R_4$' représente un groupe alkyle en C2-C20.

**15.** Le composé selon revendication 14, de formule

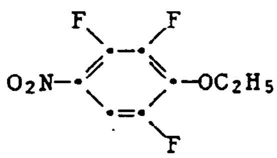

,

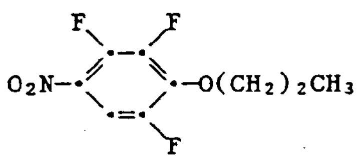

,

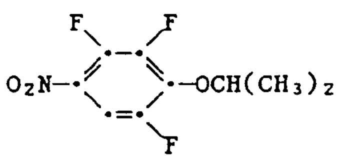

,

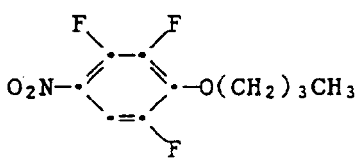

et

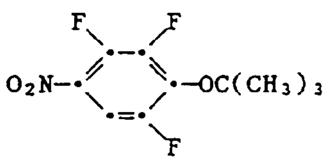

.

**Revendications pour l'Etat contractant suivant : ES**

1. Procédé de préparation d'un composé de formule I

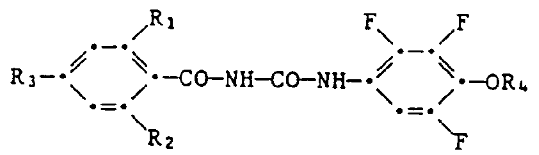

(I),

dans laquelle $R_1$ représente l'hydrogène, le fluor ou le chlore; $R_2$ représente le fluor ou le chlore; $R_3$ représente l'hydrogène ou le fluor; et $R_4$ représente un groupe alkyle en C1-C10, -C(X)F-alkyle en C1-C7 ou un groupe -C(X)F-alkyle en C1-C7 substitué par un ou plusieurs atomes d'halogènes; X représentant l'hydrogène ou un halogène, caractérisé en ce que

a) on fait réagir une aniline de formule II

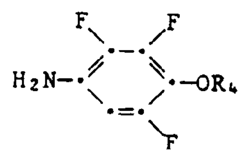

(II)

avec un isocyanate de benzoyle de formule III

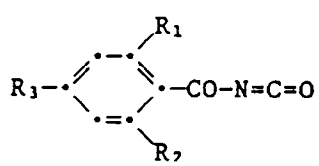

(III),

ou bien

b) on fait réagir un isocyanate de formule IV

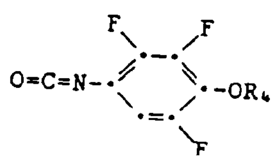

(IV)

avec un benzamide de formule V

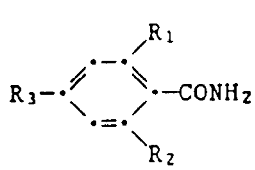

(V),

ou bien

c) on fait réagir une aniline de formule II avec un uréthanne de formule VI

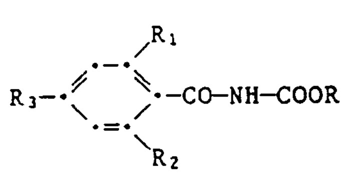

(VI)

les symboles $R_1$, $R_2$, $R_3$ et $R_4$ ayant, dans les formules II, III, IV, V et VI, les significations indiquées cidessus, et le symbole R de la formule VI représentant un groupe alkyle en C1-C8 éventuellement halogéné.

2. Procédé de préparation des composés de formule I de la revendication 1 dans laquelle $R_1$ représente l'hydrogène ou le fluor; $R_2$ représente le fluor ou le chlore; $R_3$ représente l'hydrogène ou le fluor; et $R_4$ représente un groupe alkyle en C1-C7, -C(X)F-alkyle en C1-C5 ou -C(X)F-alkyle en C1-C5 éventuellement substitué par un ou plusieurs atomes d'halogènes; X représentant l'hydrogène, le fluor ou le chlore.

3. Procédé de préparation des composés de formule I de la revendication 2 dans laquelle $R_1$ représente l'hydrogène ou le fluor; $R_2$ représente le fluor ou le chlore; $R_3$ représente l'hydrogène ou le fluor; et $R_4$ représente un groupe alkyle en C1-C3, -$CF_2$-alkyle en C1-C3 ou -$CF_2$-alkyle en C1-C3 substitué par un ou plusieurs atomes de fluor.

4. Procédé de préparation des composés selon l'une des revendications 1 à 3, de formules

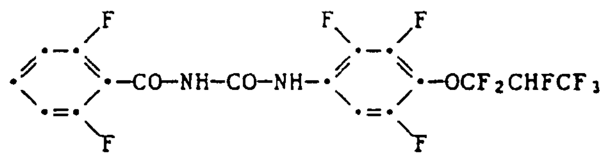 ,

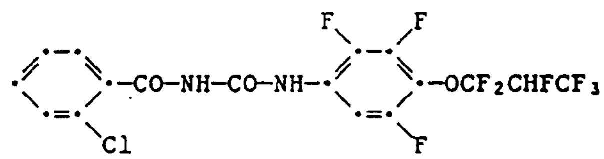 ,

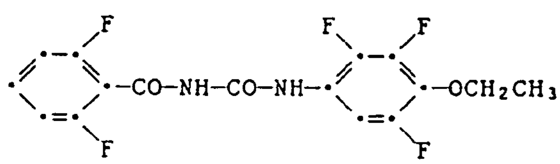 ,

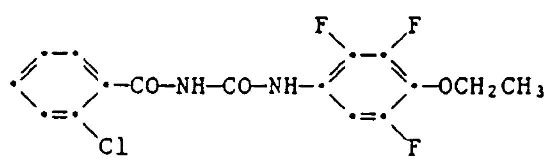 ,

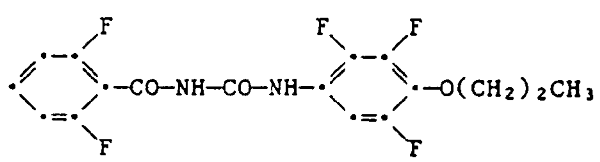 ,

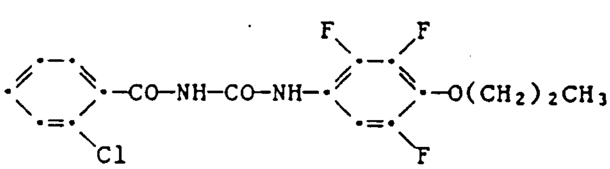 ,

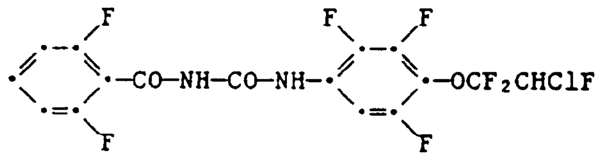
,

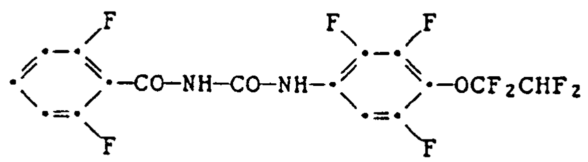
,

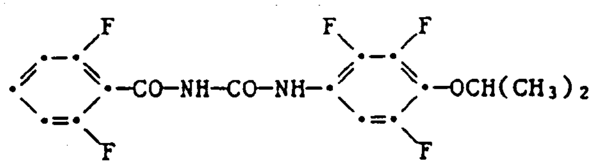
,

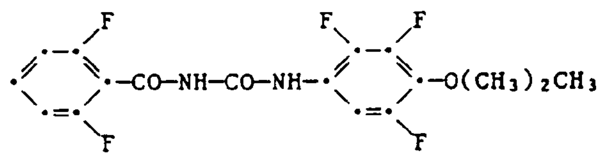
,

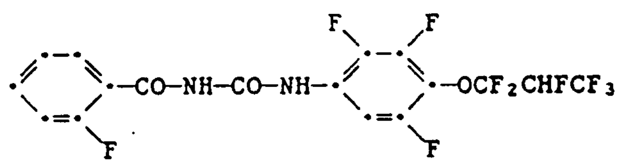
,

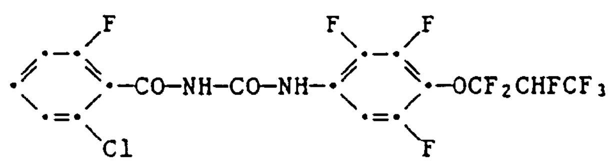
,

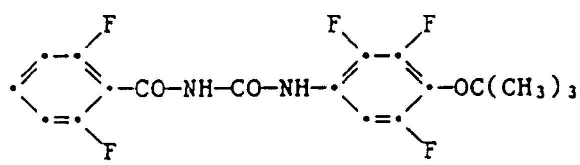
,

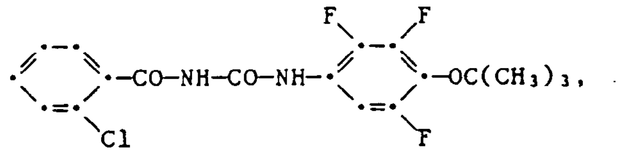

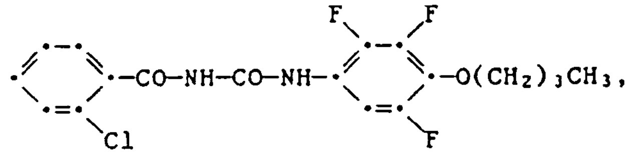

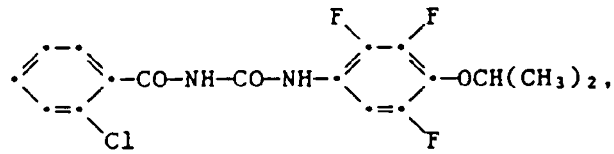

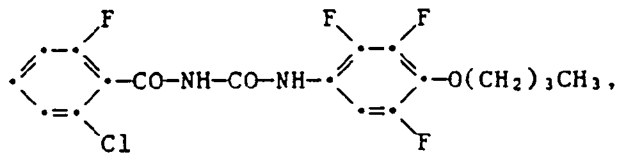

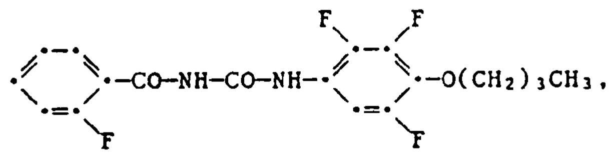

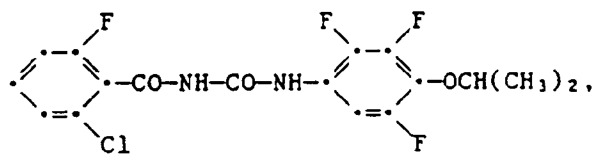

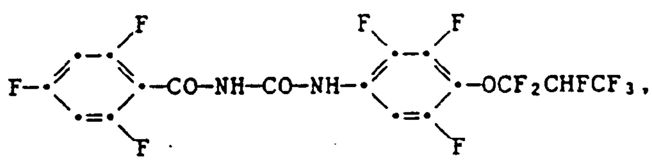

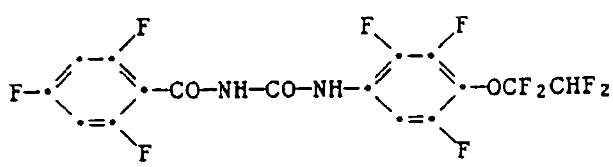

et

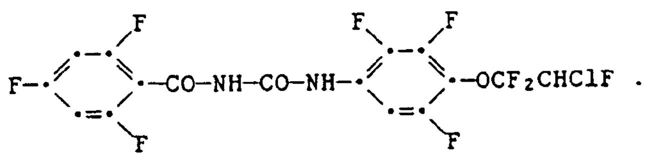

5. Produit parasiticide contenant en tant que composant actif, en quantité efficace, un composé obtenu selon revendication 1, de formule I

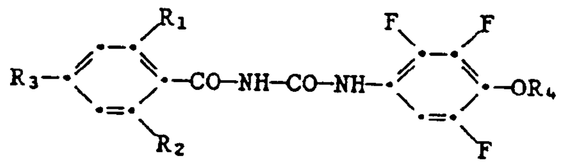

(I),

dans laquelle $R_1$ représente l'hydrogène, le fluor ou le chlore; $R_2$ représente le fluor ou le chlore; $R_3$ représente l'hydrogène ou le fluor; et $R_4$ représente un groupe alkyle en C1-C10, -C(X)F-alkyle en C1-C7 ou -C(X)F-alkyle en C1-C7 substitué par un ou plusieurs atomes d'halogènes; X représentant l'hydrogène ou un halogène, avec des véhicules et/ou additifs appropriés.

6. Procédé de préparation d'un produit selon revendication 5, caractérisé en ce que l'on mélange intimement la substance active avec le ou les produits auxiliaires.

7. Utilisation d'un composé obtenu selon revendication 1, de formule I

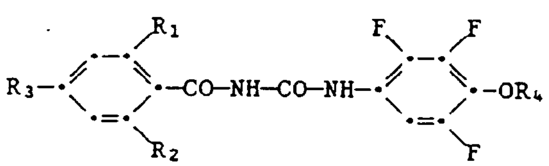

(I),

dans laquelle $R_1$ représente l'hydrogène, le fluor ou le chlore; $R_2$ représente le fluor ou le chlore; $R_3$ représente l'hydrogène ou le fluor; et $R_4$ représente un groupe alkyle en C1-C10, -C(X)F-alkyle en C1-C7 ou -C(X)F-alkyle en C1-C7 substitué par un ou plusieurs atomes d'halogènes, X représentant l'hydrogène ou un halogène, pour la lutte contre les parasites des animaux et des végétaux.

8. Utilisation selon revendication 7 d'un composé de formule I pour la lutte contre les insectes et les arachnides.

9. Utilisation selon revendication 8 d'un composé de formule I pour la lutte contre les stades larvaires des insectes nuisibles pour les végétaux.

10. Procédé pour combattre les parasites des animaux et des végétaux, caractérisé en ce que l'on met les parasites, dans leurs divers stades de développement, en contact avec un composé obtenu selon revendication 1, de formule I

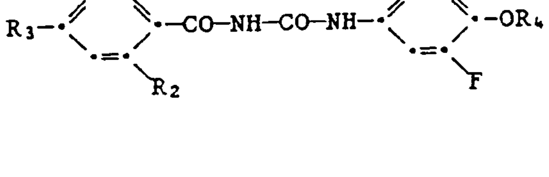

(I),

dans laquelle $R_1$ représente l'hydrogène, le fluor ou le chlore; $R_2$ représente le fluor ou le chlore; $R_3$ représente l'hydrogène ou le fluor; et $R_4$ représente un groupe alkyle en C1-C10, -C(X)F-alkyle en C1-C7 ou -C(X)F-alkyle en C1-C7 substitué par un ou plusieurs atomes d'halogènes, X représentant l'hydrogène ou un halogène.

**11.** Procédé de préparation d'un composé de formule II

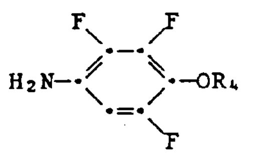

(II),

dans laquelle $R_4$ représente un groupe $-CF_2$-CHF-alkyle en C1-C6, $-CF_2$-CHF-alkyle en $C_1$-$C_6$ substitué par un ou plusieurs atomes d'halogènes, $-CF_2-CHF_2$ ou $-CF_2$-CHFCl, caractérisé en ce que l'on fait réagir un composé de formule

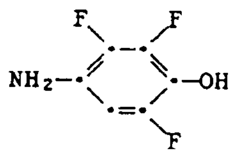

(VII)

avec $CF_2=CF_2$, $CF_2$=CFCl, $CF_2$=CF-alkyle en C1-C6 ou $CF_2$=CF-alkyle en C1-C6 substitué par un ou plusieurs atomes d'halogènes.

**12.** Procédé selon revendication 11 pour la préparation des composés de formules

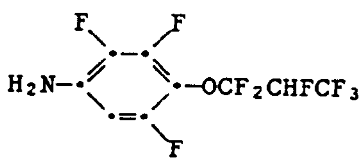

,

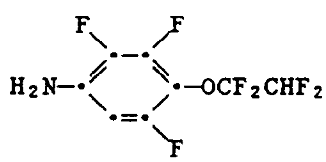

et

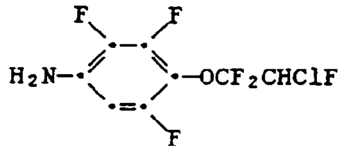

.

**13.** Procédé de préparation du composé de formule

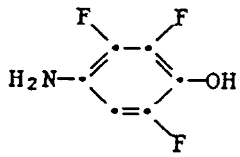

,

caractérisé en ce que l'on soumet un composé de formule

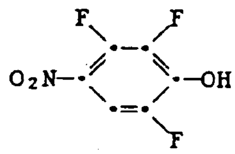

(VIII)

à hydrogénation catalytique ou réduction chimique.

**14.** Procédé de préparation d'un composé de formule IX

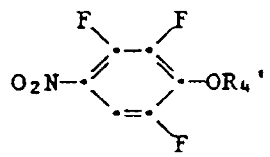

(IX),

dans laquelle $R'_4$ représente un groupe alkyle en C2-C10, caractérisé en ce que l'on fait réagir le 2,3,4,5-tétrafluoronitrobenzène avec un alcoolate métallique de formule $MetOR'_4$ dans laquelle Met représente un métal alcalino-terreux ou, de préférence, un métal alcalin, et $R'_4$ a les significations indiquées ci-dessus.

**15.** Procédé selon revendication 14, pour la préparation des composés de formules

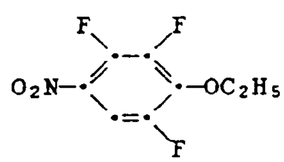

,

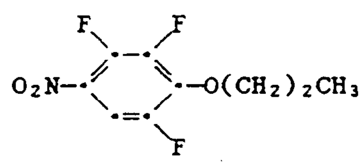

,

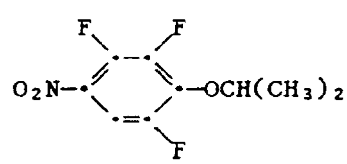

,

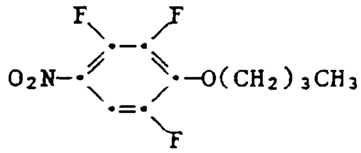

et

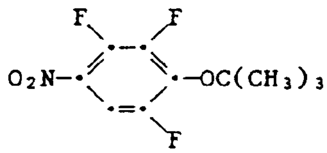

.